# EUROPEAN PATENT APPLICATION

(11) **EP 3 819 379 A1**
(43) Date of publication of application: **12.05.2021**
(21) Application number: 19829890.3
(22) Date of filing: 05.07.2019
(51) Int. Cl.: C12N 15/55, C02F 1/00, C02F 3/34, C11D 3/386, C12N 1/15, C12N 1/19, C12N 1/21, C12N 5/10, C12N 9/20

(54) **NOVEL LIPASE CAPABLE OF DECOMPOSING OIL OR FAT CONTAINING TRANS-FATTY ACID**

(30) Priority: 06.07.2018 JP 2018129504
(71) Applicant: National University Corporation Tokai National Higher Education and Research System, Nagoya-shi Aichi 464-8601 (JP)
(72) Inventor: HORI, Katsutoshi, Nagoya-shi, Aichi 464-8601 (JP)
(74) Representative: Engelhard, Markus
(86) International application number: PCT/JP2019/026848
(87) International publication number: WO 2020/009231

(57) **Abstract**

The present disclosure provides a technique for decomposing an oil or fat containing a trans-fatty acid. A novel lipase according to the present disclosure has an activity to decompose an oil or fat containing a trans-fatty acid. The lipase according to the present disclosure comprises a polypeptide comprising an amino acid sequence represented by SEQ ID NO: 4, 11, 16 or 18, or a polypeptide encoded by a nucleotide sequence represented by SEQ ID NO: 1, 7, 15 or 17, or a polypeptide having at least 70% sequence homology with the above-mentioned polypeptides. The lipase has an ability to decompose an oil or fat even at a high temperature, and is found to have excellent thermal stability. The lipase according to the present disclosure is useful for an oil treatment, such as the treatment of wastewater, a detergent, and a technique for modifying a fat or for producing an oil or fat.

SEQUENCE LISTING

<110>  NATIONAL UNIVERSITY CORPORATION NAGOYA UNIVERSITY

<120>  Novel Lipase Capable of Degrading Oil Containing Trans-Fatty
       Acids

<130>  NU002PCT

<150>  JP 2018-129504
<151>  2018-07-06

<160>  18

<170>  PatentIn version 3.5

<210>  1
<211>  963
<212>  DNA
<213>  Burkholderia arboris

<400>  1
gccgacaact acgcggcgac gcgttatccg atcatcctcg tgcacgggct caccggcacc      60

gacaagtacg ccggcgtgct cgagtactgg tacggcatcc aggaggatct gcagcagcat     120

ggcgcgaccg tctacgtcgc gaacctgtcg ggcttccaga gcgacgatgg cccgaacggg     180

cgcggcgaac agctgctcgc ctacgtgaag acggtactcg ccgcgacggg cgcgaccaag     240

gtcaatctcg tcggccacag ccagggcggg ctgacgtcgc gctatgtcgc ggccgtcgcg     300

cccgatctcg tcgcgtcggt gacgacgatc ggcacgccgc atcgcggctc cgagttcgcg     360

gatttcgtgc agagcgtgct cgcgtacgat ccgaccgggc tgtcgtcgac ggtgatcgcg     420

gcgttcgtca atgtgttcgg aatcctgacg agcagcagcc acaacacgaa ccaggatgcg     480

cttgcatcgc tgaagacgct gacgaccgct caggcggcga cctataacca gaactatccg     540

agcgcgggcc ttggtgcacc gggcagctgc cagaccggcg caccgacgga aacggtcggc     600

ggcaacacgc atctgctgta ttcgtgggcc ggcacggcga tccagccgac gctttcgctg     660

ttcggcgtga cgggtgcgaa ggacacgagc acgattccgc tcgtcgatcc cgcaaacgcg     720

ctcgacccgt cgacgctcgc gctgttcggc accggcacgg tgatgatcaa ccgtggctcg     780

ggtcagaacg acgggctcgt gtcgaagtgc agcgcgctgt acggcaaggt gctgagcacg     840

agctacaagt ggaaccacat cgacgagatc aaccagctgc tcggcgtgcg cggcgcgtat     900

gcggaagatc cggtcgcggt gatccgcacg catgcgaacc ggctgcagct cgcgggcgtg     960

taa                                                                   963


<210>  2
<211>  1056
<212>  DNA
<213>  Burkholderia arboris

<400>  2

gtggcatgtg cgatgagcgt cgcaccgttc gcggggacga ccgcgctgat gacgctcgcg 60

acgacacacg cggcgatggc ggcgaccgcg cccgccgaca actacgcggc gacgcgttat 120

ccgatcatcc tcgtgcacgg gctcaccggc accgacaagt acgccggcgt gctcgagtac 180

tggtacggca tccaggagga tctgcagcag catggcgcga ccgtctacgt cgcgaacctg 240

tcgggcttcc agagcgacga tggcccgaac gggcgcggcg aacagctgct cgcctacgtg 300

aagacggtac tcgccgcgac gggcgcgacc aaggtcaatc tcgtcggcca cagccagggc 360

gggctgacgt cgcgctatgt cgcggccgtc gcgcccgatc tcgtcgcgtc ggtgacgacg 420

atcggcacgc cgcatcgcgg ctccgagttc gcggatttcg tgcagagcgt gctcgcgtac 480

gatccgaccg ggctgtcgtc gacggtgatc gcggcgttcg tcaatgtgtt cggaatcctg 540

acgagcagca gccacaacac gaaccaggat gcgcttgcat cgctgaagac gctgacgacc 600

gctcaggcgg cgacctataa ccagaactat ccgagcgcgg gccttggtgc accgggcagc 660

tgccagaccg gcgcaccgac ggaaacggtc ggcggcaaca cgcatctgct gtattcgtgg 720

gccggcacgg cgatccagcc gacgctttcg ctgttcggcg tgacgggtgc gaaggacacg 780

agcacgattc cgctcgtcga tcccgcaaac gcgctcgacc cgtcgacgct cgcgctgttc 840

ggcaccggca cggtgatgat caaccgtggc tcgggtcaga acgacgggct cgtgtcgaag 900

tgcagcgcgc tgtacggcaa ggtgctgagc acgagctaca agtggaacca catcgacgag 960

atcaaccagc tgctcggcgt gcgcggcgcg tatgcggaag atccggtcgc ggtgatccgc 1020

acgcatgcga accggctgca gctcgcgggc gtgtaa 1056

<210> 3
<211> 1095
<212> DNA
<213> Burkholderia arboris

<400> 3
atggccagat cgatgcgttc cagggtaatg gcaggggcag tggcatgtgc gatgagcgtc 60

gcaccgttcg cggggacgac cgcgctgatg acgctcgcga cgacacacgc ggcgatggcg 120

gcgaccgcgc ccgccgacaa ctacgcggcg acgcgttatc cgatcatcct cgtgcacggg 180

ctcaccggca ccgacaagta cgccggcgtg ctcgagtact ggtacggcat ccaggaggat 240

ctgcagcagc atggcgcgac cgtctacgtc gcgaacctgt cgggcttcca gagcgacgat 300

ggcccgaacg ggcgcggcga acagctgctc gcctacgtga agacggtact cgccgcgacg 360

ggcgcgacca aggtcaatct cgtcggccac agccagggcg ggctgacgtc gcgctatgtc 420

gcggccgtcg cgcccgatct cgtcgcgtcg gtgacgacga tcggcacgcc gcatcgcggc 480

tccgagttcg cggatttcgt gcagagcgtg ctcgcgtacg atccgaccgg gctgtcgtcg 540

acggtgatcg cggcgttcgt caatgtgttc ggaatcctga cgagcagcag ccacaacacg 600

aaccaggatg cgcttgcatc gctgaagacg ctgacgaccg ctcaggcggc gacctataac 660

cagaactatc cgagcgcggg ccttggtgca ccgggcagct gccagaccgg cgcaccgacg 720

gaaacggtcg gcggcaacac gcatctgctg tattcgtggg ccggcacggc gatccagccg 780

acgctttcgc tgttcggcgt gacgggtgcg aaggacacga gcacgattcc gctcgtcgat 840

cccgcaaacg cgctcgaccc gtcgacgctc gcgctgttcg gcaccggcac ggtgatgatc 900

aaccgtggct cgggtcagaa cgacgggctc gtgtcgaagt gcagcgcgct gtacggcaag 960

gtgctgagca cgagctacaa gtggaaccac atcgacgaga tcaaccagct gctcggcgtg 1020

cgcggcgcgt atgcggaaga tccggtcgcg gtgatccgca cgcatgcgaa ccggctgcag 1080

ctcgcgggcg tgtaa 1095

<210> 4
<211> 320
<212> PRT
<213> Burkholderia arboris

<400> 4

Ala Asp Asn Tyr Ala Ala Thr Arg Tyr Pro Ile Ile Leu Val His Gly
1               5                   10                  15

Leu Thr Gly Thr Asp Lys Tyr Ala Gly Val Leu Glu Tyr Trp Tyr Gly
            20                  25                  30

Ile Gln Glu Asp Leu Gln Gln His Gly Ala Thr Val Tyr Val Ala Asn
        35                  40                  45

Leu Ser Gly Phe Gln Ser Asp Asp Gly Pro Asn Gly Arg Gly Glu Gln
    50                  55                  60

Leu Leu Ala Tyr Val Lys Thr Val Leu Ala Ala Thr Gly Ala Thr Lys
65                  70                  75                  80

Val Asn Leu Val Gly His Ser Gln Gly Gly Leu Thr Ser Arg Tyr Val
                85                  90                  95

Ala Ala Val Ala Pro Asp Leu Val Ala Ser Val Thr Thr Ile Gly Thr
            100                 105                 110

Pro His Arg Gly Ser Glu Phe Ala Asp Phe Val Gln Ser Val Leu Ala
        115                 120                 125

Tyr Asp Pro Thr Gly Leu Ser Ser Thr Val Ile Ala Ala Phe Val Asn
    130                 135                 140

Val Phe Gly Ile Leu Thr Ser Ser Ser His Asn Thr Asn Gln Asp Ala

145                 150                 155                 160

Leu Ala Ser Leu Lys Thr Leu Thr Thr Ala Gln Ala Ala Thr Tyr Asn
                165                 170                 175

Gln Asn Tyr Pro Ser Ala Gly Leu Gly Ala Pro Gly Ser Cys Gln Thr
            180                 185                 190

Gly Ala Pro Thr Glu Thr Val Gly Gly Asn Thr His Leu Leu Tyr Ser
        195                 200                 205

Trp Ala Gly Thr Ala Ile Gln Pro Thr Leu Ser Leu Phe Gly Val Thr
    210                 215                 220

Gly Ala Lys Asp Thr Ser Thr Ile Pro Leu Val Asp Pro Ala Asn Ala
225                 230                 235                 240

Leu Asp Pro Ser Thr Leu Ala Leu Phe Gly Thr Gly Thr Val Met Ile
                245                 250                 255

Asn Arg Gly Ser Gly Gln Asn Asp Gly Leu Val Ser Lys Cys Ser Ala
            260                 265                 270

Leu Tyr Gly Lys Val Leu Ser Thr Ser Tyr Lys Trp Asn His Ile Asp
        275                 280                 285

Glu Ile Asn Gln Leu Leu Gly Val Arg Gly Ala Tyr Ala Glu Asp Pro
    290                 295                 300

Val Ala Val Ile Arg Thr His Ala Asn Arg Leu Gln Leu Ala Gly Val
305                 310                 315                 320

<210>  5
<211>  351
<212>  PRT
<213>  Burkholderia arboris

<400>  5

Val Ala Cys Ala Met Ser Val Ala Pro Phe Ala Gly Thr Thr Ala Leu
1               5                   10                  15

Met Thr Leu Ala Thr Thr His Ala Ala Met Ala Ala Thr Ala Pro Ala
            20                  25                  30

Asp Asn Tyr Ala Ala Thr Arg Tyr Pro Ile Ile Leu Val His Gly Leu
        35                  40                  45

Thr Gly Thr Asp Lys Tyr Ala Gly Val Leu Glu Tyr Trp Tyr Gly Ile

50                  55                  60

Gln Glu Asp Leu Gln Gln His Gly Ala Thr Val Tyr Val Ala Asn Leu
65                  70                  75                  80

Ser Gly Phe Gln Ser Asp Asp Gly Pro Asn Gly Arg Gly Glu Gln Leu
                85                  90                  95

Leu Ala Tyr Val Lys Thr Val Leu Ala Ala Thr Gly Ala Thr Lys Val
            100                 105                 110

Asn Leu Val Gly His Ser Gln Gly Gly Leu Thr Ser Arg Tyr Val Ala
        115                 120                 125

Ala Val Ala Pro Asp Leu Val Ala Ser Val Thr Thr Ile Gly Thr Pro
    130                 135                 140

His Arg Gly Ser Glu Phe Ala Asp Phe Val Gln Ser Val Leu Ala Tyr
145                 150                 155                 160

Asp Pro Thr Gly Leu Ser Ser Thr Val Ile Ala Ala Phe Val Asn Val
                165                 170                 175

Phe Gly Ile Leu Thr Ser Ser Ser His Asn Thr Asn Gln Asp Ala Leu
            180                 185                 190

Ala Ser Leu Lys Thr Leu Thr Thr Ala Gln Ala Ala Thr Tyr Asn Gln
        195                 200                 205

Asn Tyr Pro Ser Ala Gly Leu Gly Ala Pro Gly Ser Cys Gln Thr Gly
    210                 215                 220

Ala Pro Thr Glu Thr Val Gly Gly Asn Thr His Leu Leu Tyr Ser Trp
225                 230                 235                 240

Ala Gly Thr Ala Ile Gln Pro Thr Leu Ser Leu Phe Gly Val Thr Gly
                245                 250                 255

Ala Lys Asp Thr Ser Thr Ile Pro Leu Val Asp Pro Ala Asn Ala Leu
            260                 265                 270

Asp Pro Ser Thr Leu Ala Leu Phe Gly Thr Gly Thr Val Met Ile Asn
        275                 280                 285

Arg Gly Ser Gly Gln Asn Asp Gly Leu Val Ser Lys Cys Ser Ala Leu
    290                 295                 300

Tyr Gly Lys Val Leu Ser Thr Ser Tyr Lys Trp Asn His Ile Asp Glu
305                 310                 315                 320

Ile Asn Gln Leu Leu Gly Val Arg Gly Ala Tyr Ala Glu Asp Pro Val
                325                 330                 335

Ala Val Ile Arg Thr His Ala Asn Arg Leu Gln Leu Ala Gly Val
            340                 345                 350

<210>  6
<211>  364
<212>  PRT
<213>  Burkholderia arboris

<400>  6

Met Ala Arg Ser Met Arg Ser Arg Val Met Ala Gly Ala Val Ala Cys
1               5                   10                  15

Ala Met Ser Val Ala Pro Phe Ala Gly Thr Thr Ala Leu Met Thr Leu
            20                  25                  30

Ala Thr Thr His Ala Ala Met Ala Ala Thr Ala Pro Ala Asp Asn Tyr
        35                  40                  45

Ala Ala Thr Arg Tyr Pro Ile Ile Leu Val His Gly Leu Thr Gly Thr
    50                  55                  60

Asp Lys Tyr Ala Gly Val Leu Glu Tyr Trp Tyr Gly Ile Gln Glu Asp
65                  70                  75                  80

Leu Gln Gln His Gly Ala Thr Val Tyr Val Ala Asn Leu Ser Gly Phe
                85                  90                  95

Gln Ser Asp Asp Gly Pro Asn Gly Arg Gly Glu Gln Leu Leu Ala Tyr
            100                 105                 110

Val Lys Thr Val Leu Ala Ala Thr Gly Ala Thr Lys Val Asn Leu Val
        115                 120                 125

Gly His Ser Gln Gly Gly Leu Thr Ser Arg Tyr Val Ala Ala Val Ala
    130                 135                 140

Pro Asp Leu Val Ala Ser Val Thr Thr Ile Gly Thr Pro His Arg Gly
145                 150                 155                 160

Ser Glu Phe Ala Asp Phe Val Gln Ser Val Leu Ala Tyr Asp Pro Thr
                165                 170                 175

Gly Leu Ser Ser Thr Val Ile Ala Ala Phe Val Asn Val Phe Gly Ile
            180                 185                 190

Leu Thr Ser Ser Ser His Asn Thr Asn Gln Asp Ala Leu Ala Ser Leu
        195                 200                 205

Lys Thr Leu Thr Thr Ala Gln Ala Ala Thr Tyr Asn Gln Asn Tyr Pro
    210                 215                 220

Ser Ala Gly Leu Gly Ala Pro Gly Ser Cys Gln Thr Gly Ala Pro Thr
225                 230                 235                 240

Glu Thr Val Gly Gly Asn Thr His Leu Leu Tyr Ser Trp Ala Gly Thr
                245                 250                 255

Ala Ile Gln Pro Thr Leu Ser Leu Phe Gly Val Thr Gly Ala Lys Asp
            260                 265                 270

Thr Ser Thr Ile Pro Leu Val Asp Pro Ala Asn Ala Leu Asp Pro Ser
        275                 280                 285

Thr Leu Ala Leu Phe Gly Thr Gly Thr Val Met Ile Asn Arg Gly Ser
    290                 295                 300

Gly Gln Asn Asp Gly Leu Val Ser Lys Cys Ser Ala Leu Tyr Gly Lys
305                 310                 315                 320

Val Leu Ser Thr Ser Tyr Lys Trp Asn His Ile Asp Glu Ile Asn Gln
                325                 330                 335

Leu Leu Gly Val Arg Gly Ala Tyr Ala Glu Asp Pro Val Ala Val Ile
            340                 345                 350

Arg Thr His Ala Asn Arg Leu Gln Leu Ala Gly Val
        355                 360

<210>  7
<211>  1149
<212>  DNA
<213>  Burkholderia arboris

<400>  7
aatgtcacct atcacgtcgc aggcatcccg accgccgtca ccgctcagca gttgctgtat      60

cgcaccaaca acgcgcagaa ccagcctgtc gtcaacgtga cgtcggtgat ccgcagccag     120

gtcagcaacg gccaggccat ttcgtaccag tcggcctacg attcgctgaa cccgtacgac     180

gagccgtcgc aggtgattgc cggcgaccgc gacgtgacca aggtcatcaa cgtcggcacg     240

ctgctctaca gtgcggagtc gatcccgctg tcgacgctgc tgctgctcgg ctacaacatc     300

atcgtgcccg atacggaagg ccagacggcg gacttcgcgg ccggccccga atacgggatg 360

acgacgctcg attcgatccg cgcggcgctt aatacgccgt cgacgggcct gaatccgtcg 420

agcaaggtcg cgatgatcgg ctattcgggc ggcgcaatcg cgacgaactg ggccgcgcag 480

ctcgcgccaa gttatgcgcc cgacatcaac aagcagctcg tcggcgcggc ggaaggcggc 540

gtgctggtcg atcccgcgca caacctgcgc tatgtcgacg gcagcatcgt gtggggcggc 600

gttgcggcgg ccgcgctggc cgggctgtcg cgcggctatg cgttcgacct gacgccgtat 660

ctcagcgata cgggcgtcgc cgtgttcaag gacatccaga accagtcgct tgcgtacatc 720

ctgccgaagt acacgggcct gcactggagc acgctgttca agccgcaata cgcgaacgac 780

atcaacagca tcccggcgta cgtgacgtat gcgaacaagg tgaacgcggg gctggccgca 840

tcgccgacga tcccgatgtt catcggccag ggcacggcag gcgcgctcga cggtaccttc 900

agcagccagg taggcgacgg cgtgatgctc gcgtacgacg tgcgcgccct ggcgcagaag 960

ttctgcgcca gcggcacgcc ggtcacgtac accgagtatc cgctggaaca tgcgggcgcg 1020

atcgtgccgt gggtggccgg gatgctgccc tggctctacg accgcttcaa cgggaaaacc 1080

gcgccgagca attgctggct gacgtcgctg ctgccgagca attcgctggc gcccgagacg 1140

ctgcactag 1149

<210> 8
<211> 1155
<212> DNA
<213> Burkholderia andropogonis

<400> 8
acgcgcaatg tcacctatca cgtcgcaggc atcccgaccg ccgtcaccgc tcagcagttg 60

ctgtatcgca ccaacaacgc gcagaaccag cctgtcgtca acgtgacgtc ggtgatccgc 120

agccaggtca gcaacggcca ggccatttcg taccagtcgg cctacgattc gctgaacccg 180

tacgacgagc cgtcgcaggt gattgccggc gaccgcgacg tgaccaaggt catcaacgtc 240

ggcacgctgc tctacagtgc ggagtcgatc ccgctgtcga cgctgctgct gctcggctac 300

aacatcatcg tgcccgatac ggaaggccag acggcggact tcgcggccgg ccccgaatac 360

gggatgacga cgctcgattc gatccgcgcg gcgcttaata cgccgtcgac gggcctgaat 420

ccgtcgagca aggtcgcgat gatcggctat tcgggcggcg caatcgcgac gaactgggcc 480

gcgcagctcg cgccaagtta tgcgcccgac atcaacaagc agctcgtcgg cgcggcggaa 540

ggcggcgtgc tggtcgatcc cgcgcacaac ctgcgctatg tcgacggcag catcgtgtgg 600

ggcggcgttg cggcggccgc gctggccggg ctgtcgcgcg gctatgcgtt cgacctgacg 660

ccgtatctca gcgatacggg cgtcgccgtg ttcaaggaca tccagaacca gtcgcttgcg 720

tacatcctgc cgaagtacac gggcctgcac tggagcacgc tgttcaagcc gcaatacgcg 780

aacgacatca acagcatccc ggcgtacgtg acgtatgcga acaaggtgaa cgcggggctg 840

gccgcatcgc cgacgatccc gatgttcatc ggccagggca cggcaggcgc gctcgacggt 900

accttcagca gccaggtagg cgacggcgtg atgctcgcgt acgacgtgcg cgccctggcg 960

cagaagttct gcgccagcgg cacgccggtc acgtacaccg agtatccgct ggaacatgcg 1020

ggcgcgatcg tgccgtgggt ggccgggatg ctgccctggc tctacgaccg cttcaacggg 1080

aaaaccgcgc cgagcaattg ctggctgacg tcgctgctgc cgagcaattc gctggcgccc 1140

gagacgctgc actag 1155

<210> 9
<211> 1239
<212> DNA
<213> Burkholderia arboris

<400> 9
gccgcgccga ccgtgtccga tccgttctac acgtacaccg gcgccacgcc gctggcatcg 60

attccaccgg gcacggtgct gaagacgcgc aatgtcacct atcacgtcgc aggcatcccg 120

accgccgtca ccgctcagca gttgctgtat cgcaccaaca acgcgcagaa ccagcctgtc 180

gtcaacgtga cgtcggtgat ccgcagccag gtcagcaacg gccaggccat ttcgtaccag 240

tcggcctacg attcgctgaa cccgtacgac gagccgtcgc aggtgattgc cggcgaccgc 300

gacgtgacca aggtcatcaa cgtcggcacg ctgctctaca gtgcggagtc gatcccgctg 360

tcgacgctgc tgctgctcgg ctacaacatc atcgtgcccg atacggaagg ccagacggcg 420

gacttcgcgg ccggccccga atacgggatg acgacgctcg attcgatccg cgcggcgctt 480

aatacgccgt cgacgggcct gaatccgtcg agcaaggtcg cgatgatcgg ctattcgggc 540

ggcgcaatcg cgacgaactg ggccgcgcag ctcgcgccaa gttatgcgcc cgacatcaac 600

aagcagctcg tcggcgcggc ggaaggcggc gtgctggtcg atcccgcgca caacctgcgc 660

tatgtcgacg gcagcatcgt gtggggcggc gttgcggcgg ccgcgctggc cgggctgtcg 720

cgcggctatg cgttcgacct gacgccgtat ctcagcgata cgggcgtcgc cgtgttcaag 780

gacatccaga accagtcgct tgcgtacatc ctgccgaagt acacgggcct gcactggagc 840

acgctgttca agccgcaata cgcgaacgac atcaacagca tcccggcgta cgtgacgtat 900

gcgaacaagg tgaacgcggg gctggccgca tcgccgacga tcccgatgtt catcggccag 960

ggcacggcag gcgcgctcga cggtaccttc agcagccagg taggcgacgg cgtgatgctc 1020

gcgtacgacg tgcgcgccct ggcgcagaag ttctgcgcca gcggcacgcc ggtcacgtac 1080

accgagtatc cgctggaaca tgcgggcgcg atcgtgccgt gggtggccgg gatgctgccc 1140

tggctctacg accgcttcaa cgggaaaacc gcgccgagca attgctggct gacgtcgctg 1200

ctgccgagca attcgctggc gcccgagacg ctgcactag 1239

<210>  10
<211>  1299
<212>  DNA
<213>  Burkholderia arboris

<400>  10
atgaccgtcg cggccgccgt gtgcgccgcg ctggccattg ccgcaccgtc ggccggcgcc      60

gccgcgccga ccgtgtccga tccgttctac acgtacaccg gcgccacgcc gctggcatcg     120

attccaccgg gcacggtgct gaagacgcgc aatgtcacct atcacgtcgc aggcatcccg     180

accgccgtca ccgctcagca gttgctgtat cgcaccaaca acgcgcagaa ccagcctgtc     240

gtcaacgtga cgtcggtgat ccgcagccag gtcagcaacg gccaggccat ttcgtaccag     300

tcggcctacg attcgctgaa cccgtacgac gagccgtcgc aggtgattgc cggcgaccgc     360

gacgtgacca aggtcatcaa cgtcggcacg ctgctctaca gtgcggagtc gatcccgctg     420

tcgacgctgc tgctgctcgg ctacaacatc atcgtgcccg atacggaagg ccagacggcg     480

gacttcgcgg ccggccccga atacgggatg acgacgctcg attcgatccg cgcggcgctt     540

aatacgccgt cgacgggcct gaatccgtcg agcaaggtcg cgatgatcgg ctattcgggc     600

ggcgcaatcg cgacgaactg ggccgcgcag ctcgcgccaa gttatgcgcc cgacatcaac     660

aagcagctcg tcggcgcggc ggaaggcggc gtgctggtcg atcccgcgca caacctgcgc     720

tatgtcgacg gcagcatcgt gtggggcggc gttgcggcgg ccgcgctggc cgggctgtcg     780

cgcggctatg cgttcgacct gacgccgtat ctcagcgata cgggcgtcgc cgtgttcaag     840

gacatccaga accagtcgct tgcgtacatc ctgccgaagt acacgggcct gcactggagc     900

acgctgttca agccgcaata cgcgaacgac atcaacagca tcccggcgta cgtgacgtat     960

gcgaacaagg tgaacgcggg gctggccgca tcgccgacga tcccgatgtt catcggccag    1020

ggcacggcag gcgcgctcga cggtaccttc agcagccagg taggcgacgg cgtgatgctc    1080

gcgtacgacg tgcgcgccct ggcgcagaag ttctgcgcca gcggcacgcc ggtcacgtac    1140

accgagtatc cgctggaaca tgcgggcgcg atcgtgccgt gggtggccgg gatgctgccc    1200

tggctctacg accgcttcaa cgggaaaacc gcgccgagca attgctggct gacgtcgctg    1260

ctgccgagca attcgctggc gcccgagacg ctgcactag                            1299


<210>  11
<211>  382
<212>  PRT
<213>  Burkholderia arboris

<400>  11

Asn Val Thr Tyr His Val Ala Gly Ile Pro Thr Ala Val Thr Ala Gln
1               5                   10                  15

Gln Leu Leu Tyr Arg Thr Asn Asn Ala Gln Asn Gln Pro Val Val Asn
            20                  25                  30

Val Thr Ser Val Ile Arg Ser Gln Val Ser Asn Gly Gln Ala Ile Ser
        35                  40                  45

Tyr Gln Ser Ala Tyr Asp Ser Leu Asn Pro Tyr Asp Glu Pro Ser Gln
    50                  55                  60

Val Ile Ala Gly Asp Arg Asp Val Thr Lys Val Ile Asn Val Gly Thr
65                  70                  75                  80

Leu Leu Tyr Ser Ala Glu Ser Ile Pro Leu Ser Thr Leu Leu Leu Leu
                85                  90                  95

Gly Tyr Asn Ile Ile Val Pro Asp Thr Glu Gly Gln Thr Ala Asp Phe
            100                 105                 110

Ala Ala Gly Pro Glu Tyr Gly Met Thr Thr Leu Asp Ser Ile Arg Ala
        115                 120                 125

Ala Leu Asn Thr Pro Ser Thr Gly Leu Asn Pro Ser Ser Lys Val Ala
    130                 135                 140

Met Ile Gly Tyr Ser Gly Gly Ala Ile Ala Thr Asn Trp Ala Ala Gln
145                 150                 155                 160

Leu Ala Pro Ser Tyr Ala Pro Asp Ile Asn Lys Gln Leu Val Gly Ala
                165                 170                 175

Ala Glu Gly Gly Val Leu Val Asp Pro Ala His Asn Leu Arg Tyr Val
            180                 185                 190

Asp Gly Ser Ile Val Trp Gly Gly Val Ala Ala Ala Ala Leu Ala Gly
        195                 200                 205

Leu Ser Arg Gly Tyr Ala Phe Asp Leu Thr Pro Tyr Leu Ser Asp Thr
    210                 215                 220

Gly Val Ala Val Phe Lys Asp Ile Gln Asn Gln Ser Leu Ala Tyr Ile
225                 230                 235                 240

Leu Pro Lys Tyr Thr Gly Leu His Trp Ser Thr Leu Phe Lys Pro Gln
                245                 250                 255

Tyr Ala Asn Asp Ile Asn Ser Ile Pro Ala Tyr Val Thr Tyr Ala Asn
            260                 265                 270

Lys Val Asn Ala Gly Leu Ala Ala Ser Pro Thr Ile Pro Met Phe Ile
        275                 280                 285

Gly Gln Gly Thr Ala Gly Ala Leu Asp Gly Thr Phe Ser Ser Gln Val
    290                 295                 300

Gly Asp Gly Val Met Leu Ala Tyr Asp Val Arg Ala Leu Ala Gln Lys
305                 310                 315                 320

Phe Cys Ala Ser Gly Thr Pro Val Thr Tyr Thr Glu Tyr Pro Leu Glu
                325                 330                 335

His Ala Gly Ala Ile Val Pro Trp Val Ala Gly Met Leu Pro Trp Leu
            340                 345                 350

Tyr Asp Arg Phe Asn Gly Lys Thr Ala Pro Ser Asn Cys Trp Leu Thr
        355                 360                 365

Ser Leu Leu Pro Ser Asn Ser Leu Ala Pro Glu Thr Leu His
    370                 375                 380

<210>  12
<211>  384
<212>  PRT
<213>  Burkholderia arboris

<400>  12

Thr Arg Asn Val Thr Tyr His Val Ala Gly Ile Pro Thr Ala Val Thr
1               5                   10                  15

Ala Gln Gln Leu Leu Tyr Arg Thr Asn Asn Ala Gln Asn Gln Pro Val
            20                  25                  30

Val Asn Val Thr Ser Val Ile Arg Ser Gln Val Ser Asn Gly Gln Ala
        35                  40                  45

Ile Ser Tyr Gln Ser Ala Tyr Asp Ser Leu Asn Pro Tyr Asp Glu Pro
    50                  55                  60

Ser Gln Val Ile Ala Gly Asp Arg Asp Val Thr Lys Val Ile Asn Val
65                  70                  75                  80

Gly Thr Leu Leu Tyr Ser Ala Glu Ser Ile Pro Leu Ser Thr Leu Leu
                85                  90                  95

Leu Leu Gly Tyr Asn Ile Ile Val Pro Asp Thr Glu Gly Gln Thr Ala
            100                 105                 110

Asp Phe Ala Ala Gly Pro Glu Tyr Gly Met Thr Thr Leu Asp Ser Ile
        115                 120                 125

Arg Ala Ala Leu Asn Thr Pro Ser Thr Gly Leu Asn Pro Ser Ser Lys
    130                 135                 140

Val Ala Met Ile Gly Tyr Ser Gly Gly Ala Ile Ala Thr Asn Trp Ala
145                 150                 155                 160

Ala Gln Leu Ala Pro Ser Tyr Ala Pro Asp Ile Asn Lys Gln Leu Val
                165                 170                 175

Gly Ala Ala Glu Gly Gly Val Leu Val Asp Pro Ala His Asn Leu Arg
            180                 185                 190

Tyr Val Asp Gly Ser Ile Val Trp Gly Gly Val Ala Ala Ala Ala Leu
        195                 200                 205

Ala Gly Leu Ser Arg Gly Tyr Ala Phe Asp Leu Thr Pro Tyr Leu Ser
    210                 215                 220

Asp Thr Gly Val Ala Val Phe Lys Asp Ile Gln Asn Gln Ser Leu Ala
225                 230                 235                 240

Tyr Ile Leu Pro Lys Tyr Thr Gly Leu His Trp Ser Thr Leu Phe Lys
                245                 250                 255

Pro Gln Tyr Ala Asn Asp Ile Asn Ser Ile Pro Ala Tyr Val Thr Tyr
            260                 265                 270

Ala Asn Lys Val Asn Ala Gly Leu Ala Ala Ser Pro Thr Ile Pro Met
        275                 280                 285

Phe Ile Gly Gln Gly Thr Ala Gly Ala Leu Asp Gly Thr Phe Ser Ser
    290                 295                 300

Gln Val Gly Asp Gly Val Met Leu Ala Tyr Asp Val Arg Ala Leu Ala
305                 310                 315                 320

Gln Lys Phe Cys Ala Ser Gly Thr Pro Val Thr Tyr Thr Glu Tyr Pro
                325                 330                 335

Leu Glu His Ala Gly Ala Ile Val Pro Trp Val Ala Gly Met Leu Pro
            340                 345                 350

Trp Leu Tyr Asp Arg Phe Asn Gly Lys Thr Ala Pro Ser Asn Cys Trp

355                 360                 365

Leu Thr Ser Leu Leu Pro Ser Asn Ser Leu Ala Pro Glu Thr Leu His
    370                 375                 380

<210>  13
<211>  412
<212>  PRT
<213>  Burkholderia arboris

<400>  13
   Ala Ala Pro Thr Val Ser Asp Pro Phe Tyr Thr Tyr Thr Gly Ala Thr
   1 5 10 15
   Pro Leu Ala Ser Ile Pro Pro Gly Thr Val Leu Lys Thr Arg Asn Val
   20 25 30
   Thr Tyr His Val Ala Gly Ile Pro Thr Ala Val Thr Ala Gln Gln Leu
   35 40 45
   Leu Tyr Arg Thr Asn Asn Ala Gln Asn Gln Pro Val Val Asn Val Thr
   50 55 60
   Ser Val Ile Arg Ser Gln Val Ser Asn Gly Gln Ala Ile Ser Tyr Gln
   65 70 75 80
   Ser Ala Tyr Asp Ser Leu Asn Pro Tyr Asp Glu Pro Ser Gln Val Ile
   85 90 95
   Ala Gly Asp Arg Asp Val Thr Lys Val Ile Asn Val Gly Thr Leu Leu
   100 105 110
   Tyr Ser Ala Glu Ser Ile Pro Leu Ser Thr Leu Leu Leu Leu Gly Tyr
   115 120 125
   Asn Ile Ile Val Pro Asp Thr Glu Gly Gln Thr Ala Asp Phe Ala Ala
   130 135 140
   Gly Pro Glu Tyr Gly Met Thr Thr Leu Asp Ser Ile Arg Ala Ala Leu
   145 150 155 160
   Asn Thr Pro Ser Thr Gly Leu Asn Pro Ser Ser Lys Val Ala Met Ile
   165 170 175
   Gly Tyr Ser Gly Gly Ala Ile Ala Thr Asn Trp Ala Ala Gln Leu Ala
   180 185 190
   Pro Ser Tyr Ala Pro Asp Ile Asn Lys Gln Leu Val Gly Ala Ala Glu
   195                 200                 205
   Gly Gly Val Leu Val Asp Pro Ala His Asn Leu Arg Tyr Val Asp Gly
       210                 215                 220
   Ser Ile Val Trp Gly Gly Val Ala Ala Ala Ala Leu Ala Gly Leu Ser
   225                 230                 235                 240
   Arg Gly Tyr Ala Phe Asp Leu Thr Pro Tyr Leu Ser Asp Thr Gly Val
                   245                 250                 255
   Ala Val Phe Lys Asp Ile Gln Asn Gln Ser Leu Ala Tyr Ile Leu Pro
               260                 265                 270
   Lys Tyr Thr Gly Leu His Trp Ser Thr Leu Phe Lys Pro Gln Tyr Ala
           275                 280                 285
   Asn Asp Ile Asn Ser Ile Pro Ala Tyr Val Thr Tyr Ala Asn Lys Val
       290                 295                 300
   Asn Ala Gly Leu Ala Ala Ser Pro Thr Ile Pro Met Phe Ile Gly Gln
   305                 310                 315                 320
   Gly Thr Ala Gly Ala Leu Asp Gly Thr Phe Ser Ser Gln Val Gly Asp
                   325                 330                 335
   Gly Val Met Leu Ala Tyr Asp Val Arg Ala Leu Ala Gln Lys Phe Cys
               340                 345                 350
   Ala Ser Gly Thr Pro Val Thr Tyr Thr Glu Tyr Pro Leu Glu His Ala
           355                 360                 365
   Gly Ala Ile Val Pro Trp Val Ala Gly Met Leu Pro Trp Leu Tyr Asp
       370                 375                 380
   Arg Phe Asn Gly Lys Thr Ala Pro Ser Asn Cys Trp Leu Thr Ser Leu
   385                 390                 395                 400
   Leu Pro Ser Asn Ser Leu Ala Pro Glu Thr Leu His
                   405                 410

<210>  14
<211>  432
<212>  PRT
<213>  Burkholderia arboris

<400>  14

Met Thr Val Ala Ala Ala Val Cys Ala Ala Leu Ala Ile Ala Ala Pro

1               5                   10                  15

Ser Ala Gly Ala Ala Ala Pro Thr Val Ser Asp Pro Phe Tyr Thr Tyr
            20                  25                  30

Thr Gly Ala Thr Pro Leu Ala Ser Ile Pro Pro Gly Thr Val Leu Lys
        35                  40                  45

Thr Arg Asn Val Thr Tyr His Val Ala Gly Ile Pro Thr Ala Val Thr
    50                  55                  60

Ala Gln Gln Leu Leu Tyr Arg Thr Asn Asn Ala Gln Asn Gln Pro Val
65                  70                  75                  80

Val Asn Val Thr Ser Val Ile Arg Ser Gln Val Ser Asn Gly Gln Ala
                85                  90                  95

Ile Ser Tyr Gln Ser Ala Tyr Asp Ser Leu Asn Pro Tyr Asp Glu Pro
            100                 105                 110

Ser Gln Val Ile Ala Gly Asp Arg Asp Val Thr Lys Val Ile Asn Val
        115                 120                 125

Gly Thr Leu Leu Tyr Ser Ala Glu Ser Ile Pro Leu Ser Thr Leu Leu
    130                 135                 140

Leu Leu Gly Tyr Asn Ile Ile Val Pro Asp Thr Glu Gly Gln Thr Ala
145                 150                 155                 160

Asp Phe Ala Ala Gly Pro Glu Tyr Gly Met Thr Thr Leu Asp Ser Ile
                165                 170                 175

Arg Ala Ala Leu Asn Thr Pro Ser Thr Gly Leu Asn Pro Ser Ser Lys
            180                 185                 190

Val Ala Met Ile Gly Tyr Ser Gly Gly Ala Ile Ala Thr Asn Trp Ala
        195                 200                 205

Ala Gln Leu Ala Pro Ser Tyr Ala Pro Asp Ile Asn Lys Gln Leu Val
    210                 215                 220

Gly Ala Ala Glu Gly Gly Val Leu Val Asp Pro Ala His Asn Leu Arg
225                 230                 235                 240

Tyr Val Asp Gly Ser Ile Val Trp Gly Gly Val Ala Ala Ala Ala Leu
                245                 250                 255

Ala Gly Leu Ser Arg Gly Tyr Ala Phe Asp Leu Thr Pro Tyr Leu Ser
            260                 265                 270

Asp Thr Gly Val Ala Val Phe Lys Asp Ile Gln Asn Gln Ser Leu Ala
        275                 280                 285

Tyr Ile Leu Pro Lys Tyr Thr Gly Leu His Trp Ser Thr Leu Phe Lys
    290                 295                 300

Pro Gln Tyr Ala Asn Asp Ile Asn Ser Ile Pro Ala Tyr Val Thr Tyr
305                 310                 315                 320

Ala Asn Lys Val Asn Ala Gly Leu Ala Ala Ser Pro Thr Ile Pro Met
                325                 330                 335

Phe Ile Gly Gln Gly Thr Ala Gly Ala Leu Asp Gly Thr Phe Ser Ser
            340                 345                 350

Gln Val Gly Asp Gly Val Met Leu Ala Tyr Asp Val Arg Ala Leu Ala
        355                 360                 365

Gln Lys Phe Cys Ala Ser Gly Thr Pro Val Thr Tyr Thr Glu Tyr Pro
    370                 375                 380

Leu Glu His Ala Gly Ala Ile Val Pro Trp Val Ala Gly Met Leu Pro
385                 390                 395                 400

Trp Leu Tyr Asp Arg Phe Asn Gly Lys Thr Ala Pro Ser Asn Cys Trp
                405                 410                 415

Leu Thr Ser Leu Leu Pro Ser Asn Ser Leu Ala Pro Glu Thr Leu His
            420                 425                 430

<210>  15
<211>  963
<212>  DNA
<213>  artificial sequence

<220>
<223>  Modified Sequence of the first lipase

<400>  15
tcggacgact acgcgacgac gcgttatccg atcatcctcg tgcacgggct cacgggcacc      60

gacaagtacg cgggcgtgct cgagtactgg tacggcatcc aggaagacct gcagcagcat     120

ggcgcgaccg tctacgtcgc gaacctgtcg ggcttccaga gcgatgacgg cccgaacggg     180

cgcggcgaac agctgctcgc ttacgtgaag acggtgctcg ccgcgacggg cgcgaccaag     240

gtcaatctcg tcggtcacag ccagggcggg ctcacgtcgc gttatgtcgc ggccgtcgcg     300

cccgatctcg tcgcgtcggt gacgacgatc ggcacgccgc atcgcggctc cgagttcgcc 360

gacttcgtgc agagcgtgct cgcatacgat ccgaccgggc tttcgtcgtc ggtgatcgcc 420

gcgttcgtca atgtgttcgg aatcctgacg agcagcagtc acaacacgaa ccaggacgcg 480

ctcgcgtcgc tgaagacgct gacgaccgca caggccgcca cgtacaacca gaactatccg 540

agcgcgggcc ttggcgcgcc gggcagttgc cagaccggcg caccgacgga aaccgtcggc 600

ggcaacacgc atctgctgta ttcgtgggcc ggcacggcga tccagccgac gctctccgtg 660

ttcggtgtca cgggtgcgac ggacacgagc accattccgc tcgtcgaccc ggcgaacgcg 720

ctcgatctgt cgacgctcgc gctgttcggc acgggcacgg tgatgatcaa ccgcggttcg 780

ggccagaacg acgggctcgt gtcgaagtgc agcgcgctgt acggccaggt gctgagcacg 840

agctacaagt ggaaccatat cgacgagatc aaccagttgc ttggcgtgcg cggcgcgtat 900

gcggaagatc cggtcgcggt gatccgcacg catgcgaacc ggctgaagct cgcgggcgtg 960

taa 963

<210> 16
<211> 320
<212> PRT
<213> artificial sequence

<220>
<223> Modified Sequence of the first lipase

<400> 16

Ser Asp Asp Tyr Ala Thr Thr Arg Tyr Pro Ile Ile Leu Val His Gly
1               5                   10                  15

Leu Thr Gly Thr Asp Lys Tyr Ala Gly Val Leu Glu Tyr Trp Tyr Gly
            20                  25                  30

Ile Gln Glu Asp Leu Gln Gln His Gly Ala Thr Val Tyr Val Ala Asn
        35                  40                  45

Leu Ser Gly Phe Gln Ser Asp Asp Gly Pro Asn Gly Arg Gly Glu Gln
    50                  55                  60

Leu Leu Ala Tyr Val Lys Thr Val Leu Ala Ala Thr Gly Ala Thr Lys
65                  70                  75                  80

Val Asn Leu Val Gly His Ser Gln Gly Gly Leu Thr Ser Arg Tyr Val
                85                  90                  95

Ala Ala Val Ala Pro Asp Leu Val Ala Ser Val Thr Thr Ile Gly Thr
            100                 105                 110

Pro His Arg Gly Ser Glu Phe Ala Asp Phe Val Gln Ser Val Leu Ala
        115                 120                 125

Tyr Asp Pro Thr Gly Leu Ser Ser Ser Val Ile Ala Ala Phe Val Asn
    130                 135                 140

Val Phe Gly Ile Leu Thr Ser Ser Ser His Asn Thr Asn Gln Asp Ala
145                 150                 155                 160

Leu Ala Ser Leu Lys Thr Leu Thr Thr Ala Gln Ala Ala Thr Tyr Asn
                165                 170                 175

Gln Asn Tyr Pro Ser Ala Gly Leu Gly Ala Pro Gly Ser Cys Gln Thr
            180                 185                 190

Gly Ala Pro Thr Glu Thr Val Gly Gly Asn Thr His Leu Leu Tyr Ser
        195                 200                 205

Trp Ala Gly Thr Ala Ile Gln Pro Thr Leu Ser Val Phe Gly Val Thr
    210                 215                 220

Gly Ala Thr Asp Thr Ser Thr Ile Pro Leu Val Asp Pro Ala Asn Ala
225                 230                 235                 240

Leu Asp Leu Ser Thr Leu Ala Leu Phe Gly Thr Gly Thr Val Met Ile
                245                 250                 255

Asn Arg Gly Ser Gly Gln Asn Asp Gly Leu Val Ser Lys Cys Ser Ala
            260                 265                 270

Leu Tyr Gly Gln Val Leu Ser Thr Ser Tyr Lys Trp Asn His Ile Asp
        275                 280                 285

Glu Ile Asn Gln Leu Leu Gly Val Arg Gly Ala Tyr Ala Glu Asp Pro
    290                 295                 300

Val Ala Val Ile Arg Thr His Ala Asn Arg Leu Lys Leu Ala Gly Val
305                 310                 315                 320

<210>  17
<211>  1149
<212>  DNA
<213>  artificial sequence

<220>
<223>  Modified Sequence of the second lipase

<400>  17
aacgtcacct atcacgtggc cggcattccg accgcgctga ccgcgcagca gttgctgtat        60

cgcaccaata acgcgctgaa ccagccggtc gtgaacgtga cgtcggtgat ccggagccag 120

gtcagcaacg gccgggccat ctcgtaccag tcggcttacg attcgctgaa cccgtacgac 180

gagccgtcgc aggtgatcgc cggcgatcgc gacgtcacga agatcatcaa cgtcggcacg 240

ttgctctaca gcgcggaatc gattccgctg tcgacgctgc tgctgctcgg ctacaacgtc 300

atcgtgcccg atacggaagg ccagacggcc gatttcgccg ccggccccga atacgggatg 360

acgacgctcg attcgatccg cgcggcgctc aatacgccgt cgaccggcct gagtccgtcg 420

agcaaggtcg cgatgatcgg ctattccggc ggcgcgatcg cgacgaactg ggccgcgcag 480

ctcgcgccga gctatgcacc cgagatcaac aggcagctcg tcggcgcggc ggagggcggc 540

gtgctggtcg accccgcgca caacctgcgc tatgtcgacg gcagcatcgt gtggggcggc 600

gtggccgcgg ccgcgctggc cgggctgtcg cgcggctacg gcttcgacct gacgccctat 660

ctcagcgata ccggcgtcgc cgtgttcaac gacatccaga gccagtcgct cgcgtacatc 720

ctgccgaaat acacggggct gcgctggggc acgctgttca agccgcaata cgcgaacgac 780

atcaacagca ttcccgcgta cgtgacgtat gccaacaagg tgaatgccgg gctggccgca 840

tcgccgacga tcccgatgtt catcggccag ggcacggcgg gcgcgctgga cggcaccttc 900

agcagccagg tgggtgacgg cgtgatgctc gcgtatgacg tgcgcgccct tgcgcagaag 960

ttctgcgcca gcggcacgcc ggtcacgtac aacgagtatc cgctcgagca tgcgggggcg 1020

atcgtgccgt gggtggccgg catgctgccg tggctctacg accgtttcaa cgggaaagcc 1080

gcgccgagca attgctggct gacgtcgctg ctgccgagca attcgctggc gcccgaaacg 1140

ctgcattag 1149

<210> 18
<211> 382
<212> PRT
<213> artificial sequence

<220>
<223> Modified Sequence of the second lipase

<400> 18

Asn Val Thr Tyr His Val Ala Gly Ile Pro Thr Ala Leu Thr Ala Gln
1               5                   10                  15

Gln Leu Leu Tyr Arg Thr Asn Asn Ala Leu Asn Gln Pro Val Val Asn
            20                  25                  30

Val Thr Ser Val Ile Arg Ser Gln Val Ser Asn Gly Arg Ala Ile Ser
        35                  40                  45

Tyr Gln Ser Ala Tyr Asp Ser Leu Asn Pro Tyr Asp Glu Pro Ser Gln
    50                  55                  60

Val Ile Ala Gly Asp Arg Asp Val Thr Lys Ile Ile Asn Val Gly Thr
65                  70                  75                  80

Leu Leu Tyr Ser Ala Glu Ser Ile Pro Leu Ser Thr Leu Leu Leu Leu
                85                  90                  95

Gly Tyr Asn Val Ile Val Pro Asp Thr Glu Gly Gln Thr Ala Asp Phe
            100                 105                 110

Ala Ala Gly Pro Glu Tyr Gly Met Thr Thr Leu Asp Ser Ile Arg Ala
        115                 120                 125

Ala Leu Asn Thr Pro Ser Thr Gly Leu Ser Pro Ser Ser Lys Val Ala
    130                 135                 140

Met Ile Gly Tyr Ser Gly Gly Ala Ile Ala Thr Asn Trp Ala Ala Gln
145                 150                 155                 160

Leu Ala Pro Ser Tyr Ala Pro Glu Ile Asn Arg Gln Leu Val Gly Ala
                165                 170                 175

Ala Glu Gly Gly Val Leu Val Asp Pro Ala His Asn Leu Arg Tyr Val
            180                 185                 190

Asp Gly Ser Ile Val Trp Gly Gly Val Ala Ala Ala Ala Leu Ala Gly
        195                 200                 205

Leu Ser Arg Gly Tyr Gly Phe Asp Leu Thr Pro Tyr Leu Ser Asp Thr
    210                 215                 220

Gly Val Ala Val Phe Asn Asp Ile Gln Ser Gln Ser Leu Ala Tyr Ile
225                 230                 235                 240

Leu Pro Lys Tyr Thr Gly Leu Arg Trp Gly Thr Leu Phe Lys Pro Gln
                245                 250                 255

Tyr Ala Asn Asp Ile Asn Ser Ile Pro Ala Tyr Val Thr Tyr Ala Asn
            260                 265                 270

Lys Val Asn Ala Gly Leu Ala Ala Ser Pro Thr Ile Pro Met Phe Ile
        275                 280                 285

Gly Gln Gly Thr Ala Gly Ala Leu Asp Gly Thr Phe Ser Ser Gln Val
    290                 295                 300

Gly Asp Gly Val Met Leu Ala Tyr Asp Val Arg Ala Leu Ala Gln Lys

305                 310                 315                 320

Phe Cys Ala Ser Gly Thr Pro Val Thr Tyr Asn Glu Tyr Pro Leu Glu
                325                 330                 335

His Ala Gly Ala Ile Val Pro Trp Val Ala Gly Met Leu Pro Trp Leu
            340                 345                 350

Tyr Asp Arg Phe Asn Gly Lys Ala Ala Pro Ser Asn Cys Trp Leu Thr
        355                 360                 365

Ser Leu Leu Pro Ser Asn Ser Leu Ala Pro Glu Thr Leu His
    370                 375                 380

## Description

### [Technical Field]

The present disclosure relates to a novel lipase that decomposes trans fatty acid-containing oil and fat and applications thereof (e.g., wastewater treatment and oil treatment). In another aspect, the present disclosure relates to a novel lipase that decomposes oil even at a high temperature and applications thereof.

### [Background Art]

Representative stains that are generated at households, restaurant industry, industrial facilities and the like include oil stains. Oil stains are stains that are difficult to clean, generated in a household kitchen, sink, commercial kitchen, pipe, drainage system, or ventilator, or during an occasion such as laundry. Since oil stains can be a source of foul odor or pests and result in environmental contamination, there is an earnest demand for the establishment of a revolutionary technology in oil treatment, from the perspective of both public health and environmental aspects.

A grease trap, which is a treatment facility for removing oil content in kitchen wastewater produced by the restaurant industry by solid-liquid separation, is a source of foul odor or pests, and entails cost and labor associated with maintenance such as collection and transport of the separated oil and cleaning. In view of this, there is an earnest demand for the establishment of a revolutionary technology that would eliminate oil within a grease trap from industries including the restaurant industry.

### [Summary of Invention]

### [Solution to Problem]

As a result of diligent studies, the inventors found a lipase that decomposes trans fatty acid-containing oil and fat. In some aspects, this lipase was found to have the ability to decompose oil and fat even at a high temperature and have excellent thermal stability. The present disclosure also relates to applications of the lipase of the present disclosure, such as oil treatment.

Therefore, the present disclosure provides the following.

### (Item 1)

A polypeptide, which is
(a) a polypeptide comprising the amino acid sequence set forth in SEQ ID NO: 4, 11, 16, or 18;
(b) a polypeptide having biological activity, comprising an amino acid sequence comprising one or more amino acid substitutions, additions, deletions, or a combination thereof in the amino acid sequence of (a);
(c) a polypeptide having biological activity, having at least 70% sequence identity to the amino acid sequence of (a) or (b);
(d) a polypeptide comprising an amino acid sequence encoded by the nucleic acid sequence set forth in SEQ ID NO: 1, 7, 15, or 17;
(e) a polypeptide having biological activity, encoded by a nucleic acid sequence comprising one or more nucleotide substitutions, additions, deletions, or a combination thereof in the nucleic acid sequence of (d);
(f) a polypeptide having biological activity, encoded by a nucleic acid sequence having at least 70% sequence identity to the nucleic acid sequence of (d) or (e);
(g) a polypeptide having biological activity and encoded by a nucleic acid sequence that hybridizes with a polynucleotide comprising the nucleic acid sequence of any one of (d) to (f) or a complementary sequence thereof under a stringent condition;
(h) a polypeptide having biological activity, encoded by an allelic mutant of the nucleic acid sequence of any one of (d) to (g); or
(i) a polypeptide comprising a fragment of the amino acid sequence of (a) to (h).

### (Item 2)

The polypeptide of item 1, wherein the biological activity is an ability associated with assimilation of trans fatty acid-containing oil and fat or an ability to decompose trans fatty acid-containing oil and fat.

### (Item 3)

The polypeptide of item 1 or 2, wherein 45 to 70°C is an optimal temperature for an ability to decompose oil and fat.

### (Item 4)

The polypeptide of any one of items 1 to 3, wherein 35 to 55°C is an optimal temperature for an ability to decompose oil and fat.

### (Item 5)

The polypeptide of any one of items 1 to 4, having thermal stability at 65°C or greater, and preferably at 85°C or less.

### (Item 6)

The polypeptide of any one of items 1 to 5, having thermal stability at 75°C or greater, and preferably at 80°C or less.

### (Item 7)

The polypeptide of any one of items 1 to 6, having an ability to decompose oil and fat at 15°C.

### (Item 8)

The polypeptide of any one of items 1 to 7, which is a polypeptide derived from Burkholderia arboris.

### (Item 9)

A polynucleotide, which is
(A) a polynucleotide comprising the nucleic acid sequence set forth in SEQ ID NO: 1, 7, 15, or 17;
(B) a polynucleotide comprising a nucleic acid sequence comprising one or more nucleotide substitutions, additions, deletions, or a combination thereof in the nucleic acid sequence of (A);
(C) a polynucleotide encoding a polypeptide having biological activity, comprising a nucleic acid sequence having at least 70% sequence identity to the nucleic acid sequence of (A) or (B);
(D) a polynucleotide encoding a polypeptide having biological activity and comprising a nucleic acid sequence that hybridizes with a polynucleotide comprising the nucleic acid sequence of any one of (A) to (C) or a complementary sequence thereof under a stringent condition;
(E) a polynucleotide, which is an allelic mutant of the nucleic acid sequence of any one of (A) to (D), encoding a polypeptide having biological activity;
(F) a polynucleotide encoding a polypeptide comprising the amino acid sequence set forth in SEQ ID NO: 4, 11, 16, or 18;
(G) a polynucleotide encoding a polypeptide having biological activity, comprising an amino acid sequence comprising one or more amino acid substitutions, additions, deletions, or a combination thereof in the amino acid sequence of (F);
(H) a polynucleotide encoding a polypeptide having biological activity, having at least 70% sequence identity to the amino acid sequence of (F) or (G); or (I) a polynucleotide comprising a fragment of the nucleic acid sequence of (F) to (H).

### (Item 10)

The polynucleotide of item 9, wherein the biological activity is an ability associated with assimilation of trans fatty acid-containing oil and fat or an ability to decompose trans fatty acid-containing oil and fat.

### (Item 11)

The polynucleotide of item 9 or 10, encoding a polypeptide for which 45 to 70°C is an optimal temperature for an ability to decompose oil and fat.

### (Item 12)

The polynucleotide of any one of items 9 to 11, encoding a polypeptide for which 35 to 55°C is an optimal temperature for an ability to decompose oil and fat.

### (Item 13)

The polynucleotide of any one of items 9 to 12, encoding a polypeptide having thermal stability at 65°C or greater, and preferably at 85°C or less.

### (Item 14)

The polynucleotide of any one of items 9 to 13, encoding a polypeptide having thermal stability at 75°C or greater, and preferably at 80°C or less.

### (Item 15)

The polynucleotide of any one of items 9 to 14, wherein the polynucleotide encodes a polypeptide having an ability to decompose oil and fat at 15°C.

### (Item 16)

The polynucleotide of any one of items 9 to 15, which is a polynucleotide derived from Burkholderia arboris.

### (Item 17)

A cell or a cell-free expression system comprising the polynucleotide of any one of items 9 to 16.

### (Item 18)

An oil decomposing agent comprising the polypeptide of any one of items 1 to 8, a cell or a cell-free expression system comprising the polynucleotide of any one of items 9 to 16, or the cell or cell-free expression system of item 17.

### (Item 19)

The oil decomposing agent of item 18, comprising an additional oil treating component.

### (Item 20)

A kit for decomposing oil, comprising the polypeptide of any one of items 1 to 8, a cell or a cell-free expression system comprising the polynucleotide of any one of items 9 to 16, the cell or cell-free expression system of item 17, or the oil decomposing agent of item 18, and an additional oil treating component.

### (Item 21)

An oil decomposing and removing method comprising causing the polypeptide of any one of items 1 to 8, a cell or a cell-free expression system comprising the polynucleotide of any one of items 9 to 16, the cell or cell-free expression system of item 17, or the oil decomposing agent of item 18 or 19 to act on a subject of treatment.

### (Item 22)

The method of item 21, wherein the subject of treatment comprises trans fatty acid or trans fatty acid-containing oil and fat.

### (Item 23)

A detergent comprising the polypeptide of any one of items 1 to 8, a cell or a cell-free expression system comprising the polynucleotide of any one of items 9 to 16, or the cell or cell-free expression system of item 17.

### (Item 24)

Use of the polypeptide of any one of items 1 to 8, a cell or a cell-free expression system comprising the polynucleotide of any one of items 9 to 16, the cell or cell-free expression system of item 17, or the oil decomposing agent of item 18 or 19 in a technology for fat modification or oil and fat production (transesterification or the like).

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

Treatment of trans fatty acid-containing oil and fat, which was difficult to remove with conventional art, has been facilitated with the use of the lipase of the present disclosure. The lipase enables oil and fat removal or treatment of oil even under a high temperature or low temperature environment, where use of conventional microorganisms or enzymes was difficult. Therefore, the novel lipase of the present disclosure is useful in technical fields of detergents, leather industry, food industry, cleanup of an environmental contamination due to oil and fat, food waste treatment, composting treatment, waste treatment such as wastewater treatment, composting, pharmaceutical products such as digestion agents, and cosmetic products for oily skin, and the like.

### [Brief Description of Drawings]

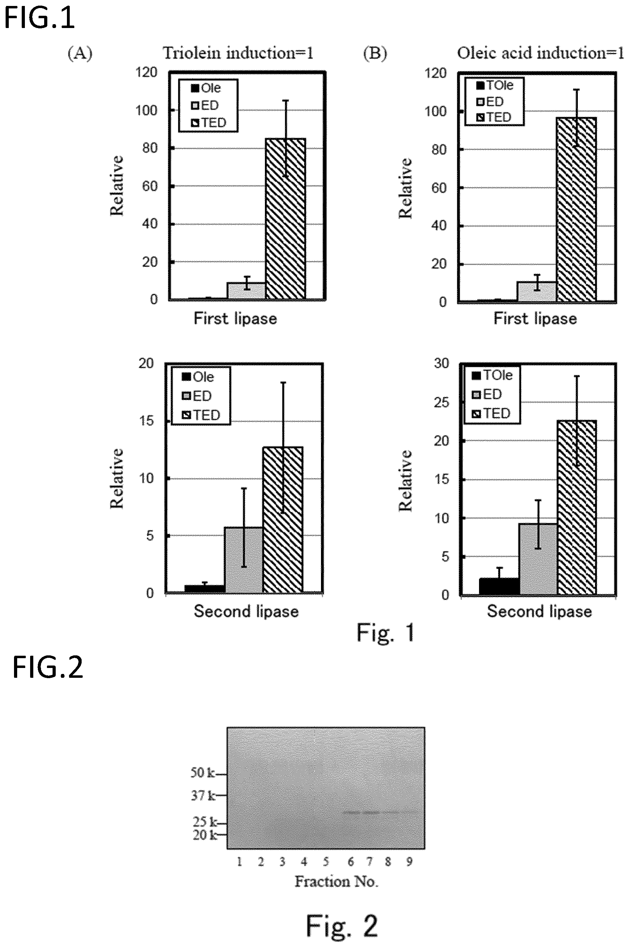

[Figure 1] Figure 1 shows an expression level of a gene encoding the lipase of the present disclosure when oil and fat was added. After culturing a KH-1 strain for 6 hours at 28°C in an inorganic salt medium comprising triolein, oleic acid, elaidic acid, or trielaidin, RNA was extracted from the KH-1 strain, and the expression level of the lipase gene of the present disclosure was quantified by quantitative RT-PCR. Ole indicates culture with oleic acid, TOle indicates culture with triolein, ED indicates culture with elaidic acid, and TED indicates results in culture with trielaidin. The expression levels are indicated as a relative value obtained from normalizing the levels with the amount of expression of an rpoD gene, and setting the expression level in culture with oleic acid or triolein to 1. (A) shows the relative value to the expression level with triolein at 1, and (B) shows the relative value to the expression level with oleic acid at 1. The graphs in the top row show the expression levels of a first lipase gene with a representative sequence, and the graphs in the bottom row show the expression levels of a second lipase gene with a representative sequence. The error bar indicates the standard deviation.
[Figure 2] Figure 2 shows purification of the first lipase of the present disclosure with a representative sequence from KH-1 stain culture supernatant cultured at 28°C. The KH-1 strain culture supernatant cultured at 28°C was purified by hydrophobic column chromatography. The results of analysis using CBB staining of an elution fraction are shown. The numbers of the eluted fractions are shown from the left. The left side indicates the molecular weight.

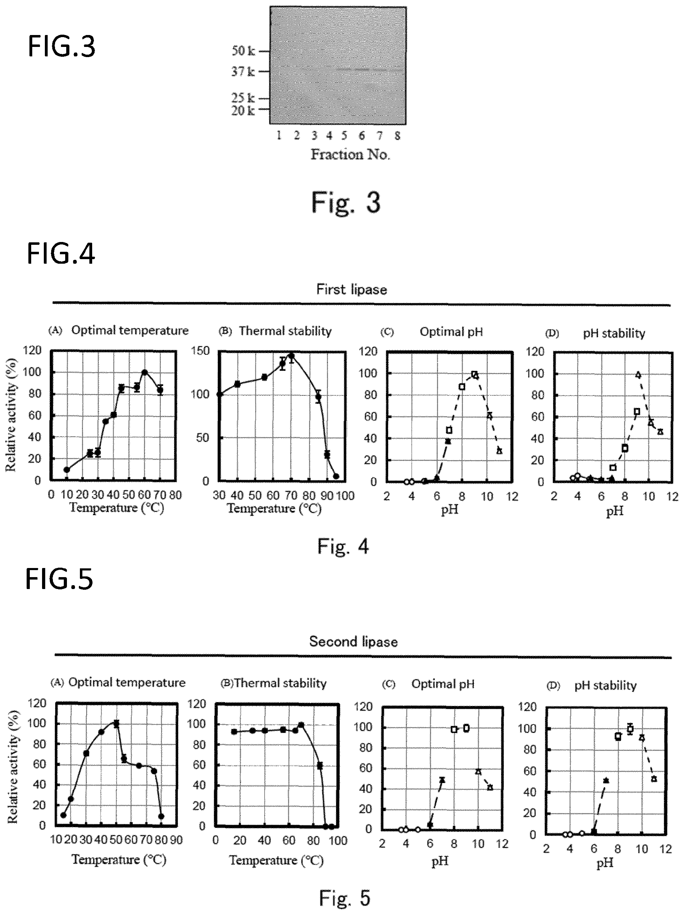

[Figure 3] Figure 3 shows purification of the second lipase of the present disclosure with a representative sequence from KH-1 stain culture supernatant cultured at 15°C. The culture supernatant of a KH-1 strain cultured at 15°C was purified by hydrophobic column chromatography. The results of analysis using CBB staining on an elution fraction are shown. The numbers of the eluted fractions are shown from the left. The left side indicates the molecular weight.
[Figure 4] Figure 4 shows, from the left, the optimal temperature (A), thermal stability (B), optimal pH (C), and pH stability (D) for the first lipase with a representative sequence. The vertical axes of the graphs for (A) to (D) indicate relative lipase activity. The horizontal axes of (A) and (B) indicate temperature, and the horizontal axes in (C) and (D) indicate pH. In (C) and (D), "o" indicates results of analysis in the range of pH 3.0 to 5.0 by using acetic acid buffer, **"▲"** indicates results of analysis in the range of pH 5.0 to 7.0 by using sodium phosphate buffer, "□" indicates results of analysis in the range of pH 7.0 to 9.0 by using Tris-HCl buffer, and **"Δ"** indicates results of analysis in the range of pH 9.0 to 11.0 by using CAPS buffer. The error bar indicates the standard deviation.
[Figure 5] Figure **5** shows, from the left, the optimal temperature (A), thermal stability (B), optimal pH (C), and pH stability (D) for the second lipase with a representative sequence. The vertical axes of the graphs for (A) to (D) indicate relative lipase activity. The horizontal axes of (A) and (B) indicate temperature, and the horizontal axes in (C) and (D) indicate pH. In (C) and (D), "o" indicates results of analysis in the range of pH 3.0 to 5.0 by using acetic acid buffer, "**▲"** indicates results of analysis in the range of pH 5.0 to 7.0 by using sodium phosphate buffer, "□" indicates results of analysis in the range of pH 7.0 to 9.0 by using Tris-HCl buffer, and **"Δ"** indicates results of analysis in the range of pH 9.0 to 11.0 by CAPS buffer. The error bar indicates the standard deviation.

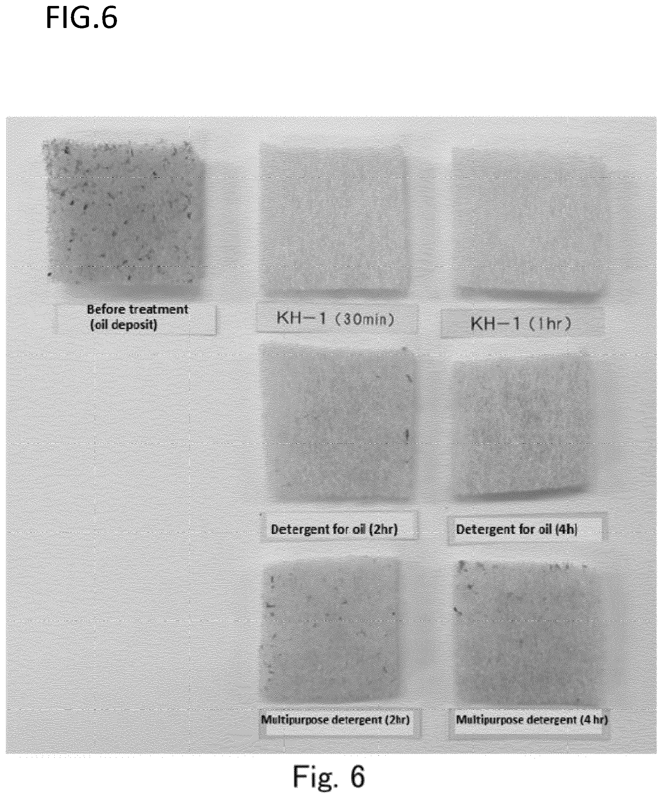

[Figure 6] Figure **6** shows results of comparing activities of decomposing stain on a ventilator of culture supernatant comprising the first and second lipases with a representative sequence with a detergent for oil and multipurpose detergent. The top left filter is a filter with oil adhering thereto, prior to treatment. The center and right sides on the top row show filters that were soaked for 30 minutes and 1 hour in KH-1 culture supernatant, respectively. The center and right sides on the middle row show filters soaked for 2 hours and 4 hours, respectively, in a detergent for oil. The center and right sides on the bottom row show filters soaked for 2 hours and 4 hours, respectively, in a multipurpose detergent.

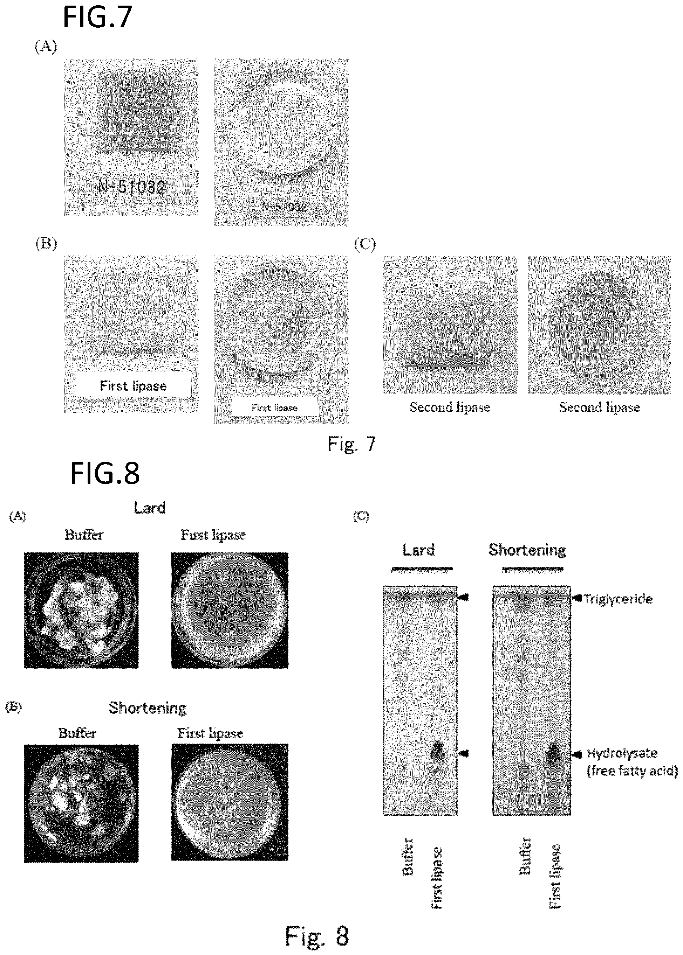

[Figure 7] Figure **7** shows comparison of activities of decomposing stain on a ventilator of the first and second lipases with a representative sequence with a Novozym® 51032 lipase (Novozymes). (A) The left side shows a picture of a ventilator filter after being soaked in Novozym 51032 lipase (N-51032) for 30 minutes, and the right side shows a picture of a solution of Novozym 51032 lipase after the ventilator filter was soaked. (B) The left side shows a picture of a ventilator filter after being soaked in the first lipase with a representative sequence for 30 minutes, and the right side shows a picture of a solution of the first lipase with a representative sequence after the ventilator filter was soaked. (C) The left side shows a picture of a ventilator filter after being soaked in the second lipase with a representative sequence for 30 minutes, and the right side shows a picture of a solution of the second lipase with a representative sequence after the ventilator filter was soaked.
[Figure 8] Figure **8** shows activity of decomposing lard and shortening by the first lipase with a representative sequence. The first lipase with a representative sequence was purified by hydrophobic column chromatography from a KH-1 strain cultured at 28°C. (A) shows lard incubated with buffer or the first lipase with a representative sequence. (B) shows shortening incubated with buffer or the first lipase with a representative sequence. (C) shows results of separating extracted oil and fat by thin-layer chromatography.

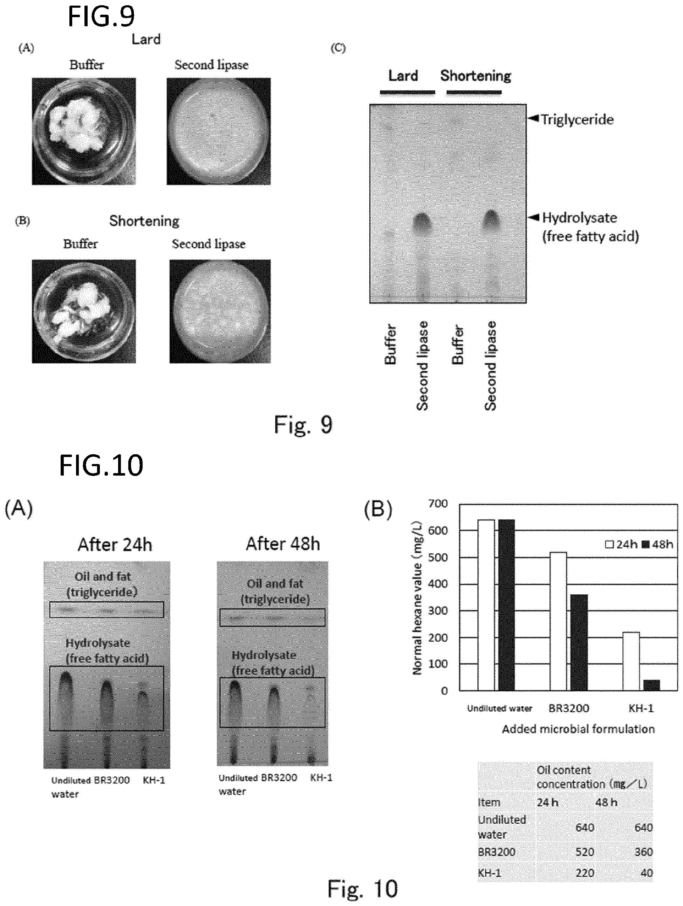

[Figure 9] Figure **9** shows activity of decomposing lard and shortening by the second lipase with a representative sequence. The second lipase with a representative sequence was purified by hydrophobic column chromatography from a KH-1 strain cultured at 15°C. (A) shows lard incubated with buffer or the second lipase with a representative sequence. (B) shows shortening incubated with buffer or the second lipase with a representative sequence. (C) shows results of separating extracted oil and fat by thin-layer chromatography.
[Figure 10] Figure **10** shows a test of treating trans fatty acid-containing wastewater with a KH-1 strain and BR3200 (BioRemove 3200, Novozymes). (A) shows results of separating oil and fat in wastewater by thin-layer chromatography after providing trans fatty acid-containing wastewater and culturing the KH-1 strain and BR3200 for 24 hours or 48 hours. (B) shows the concentrations of oil content in wastewater after culturing the KH-1 strain and BR3200.

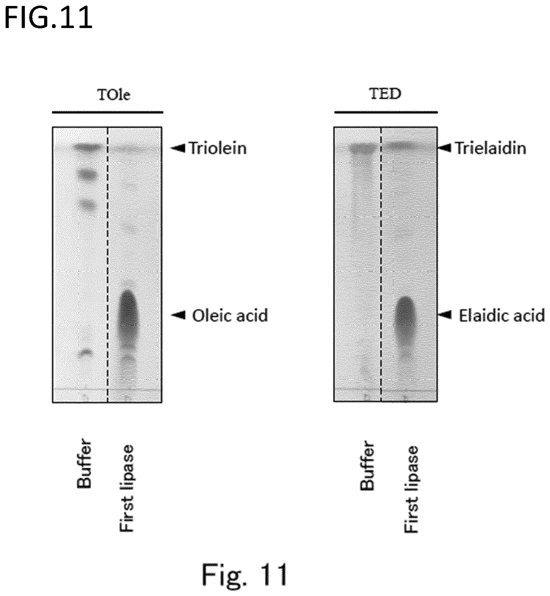

[Figure 11] Figure **11** shows decomposition of triolein and trielaidin under a 37°C condition by the first lipase with a representative sequence. The left side shows the decomposition of triolein, and the right side shows decomposition of trielaidin. TOle indicates triolein, and TED indicates trielaidin. Triglyceride (triolein or trielaidin) signals are shown on the top side of each plate, and free fatty acid (oleic acid or elaidic acid) signals are shown on the bottom side. Results of treatment with only enzyme-free buffer are also shown as a control.

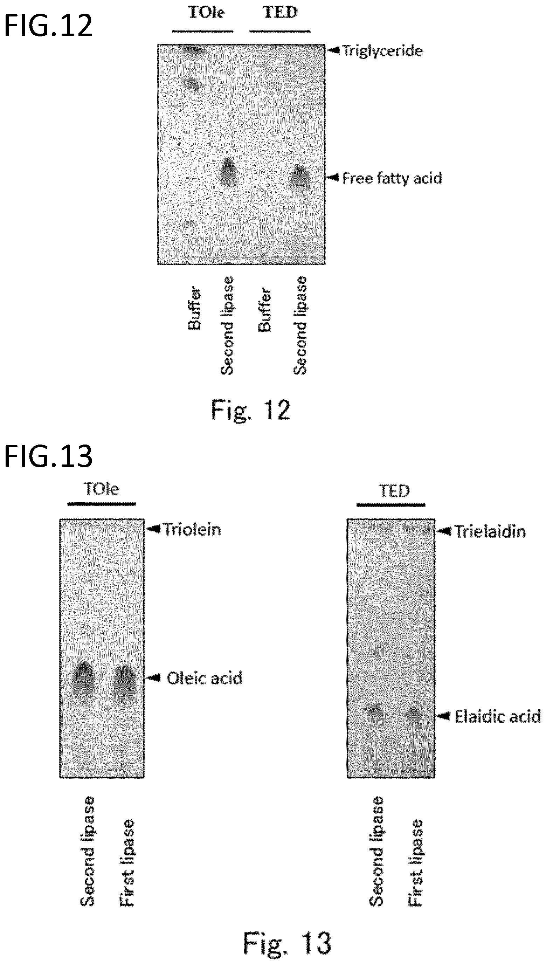

[Figure 12] Figure **12** shows decomposition of triolein and trielaidin under a 37°C condition by the second lipase with a representative sequence. The left side shows the decomposition of triolein, and the right side shows decomposition of trielaidin. TOle indicates triolein, and TED indicates trielaidin. Triglyceride signals are shown on the top side of each plate, and free fatty acid signals are shown on the bottom side. Results of treatment with only enzyme-free buffer are also shown as a control.
[Figure 13] Figure **13** shows decomposition of triolein and trielaidin under a 15°C condition by the lipase of the present disclosure. The first lipase with a representative sequence and the second lipase with a representative sequence were purified from KH-1 strain culture supernatant with a Butyl sepharose column, mixed with trielaidin or triolein and treated for 24 hours at 15°C. The left side shows treatment of triolein, and the right side shows treatment of trielaidin. In each plate, the left side shows treatment with a solution comprising the second lipase with a representative sequence, and the right side shows treatment with a solution comprising the first lipase with a representative sequence. The top side of each plate shows triglyceride (triolein or trielaidin) signals, and the bottom side shows free fatty acid (oleic acid or elaidic acid) signals.

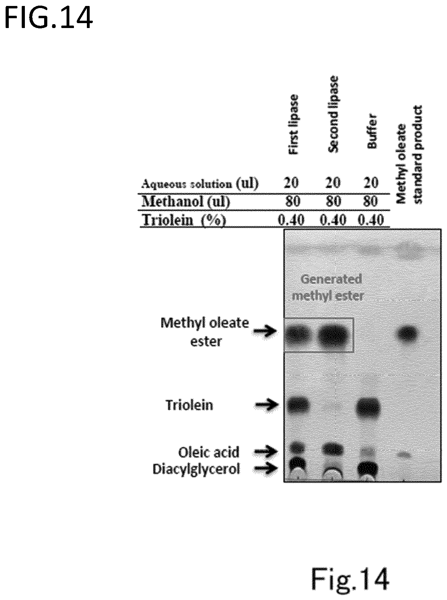

[Figure 14] Figure **14** shows fat modification of triolein by the first and second lipases of the present disclosure. The figure shows results of analyzing reaction mixtures of the first and second lipases with a representative sequence or buffer alone with methanol and triolein, and a methyl oleate standard product by TLC anlaysis. Each signal corresponds to, from the top in order, methyl oleate ester, triolein, oleic acid, and diacylglycerol.

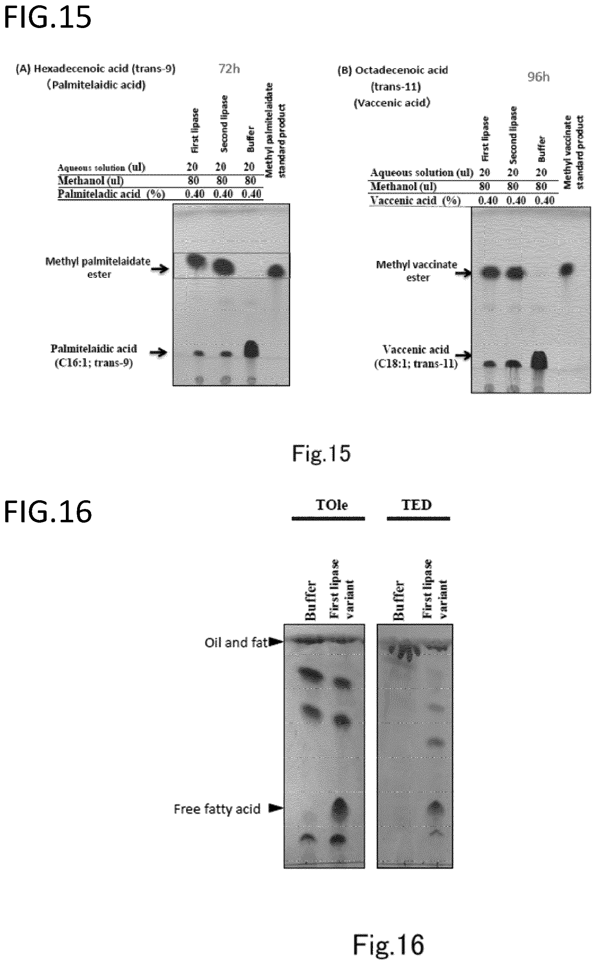

[Figure 15] Figure **15** shows fat modification of trans fatty acid by the first and second lipases of the present disclosure. (A) shows results of analyzing reaction mixtures of the first and second lipases with a representative sequence or buffer alone with methanol and palmitelaidic acid, and methyl palmitelaidate standard product by TLC anlaysis. Each signal corresponds to, from the top in order, methyl palmitelaidate ester and palmitelaidic acid. (B) shows results of analyzing reaction mixtures of the first and second lipases with a representative sequence or buffer alone with methanol and vaccenic acid, and methyl vaccinate standard product by TLC anlaysis. Each signal corresponds to, from the top in order, methyl vaccenate ester and vaccenic acid.
[Figure 16] Figure **16** shows the activity to decompose oil and fat of a variant of the first lipase of the invention. The left side shows decomposition of triolein (TOle), and the right side shows decomposition of trielaidin (TED). In each photograph, the left lane shows a result of treatment with buffer alone, and the right lane shows a result of treatment with a variant of the first lipase. In each picture, the top arrow corresponds to the oil and fat that is not decomposed, and the bottom arrow corresponds to free fatty acid.

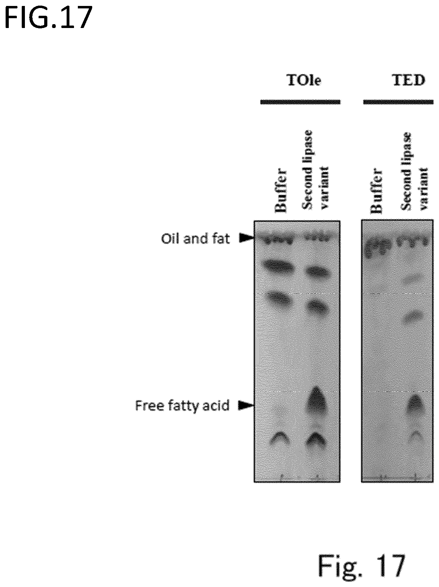

[Figure 17] Figure **17** shows the activity to decompose oil and fat of a variant of the second lipase of the invention. The left side shows decomposition of triolein (TOle), and the right side shows decomposition of trielaidin (TED). In each photograph, the left lane shows a result of treatment with buffer alone, and the right lane shows a result of treatment with a variant of the second lipase. In each picture, the top arrow corresponds to the oil and fat that is not decomposed, and the bottom arrow corresponds to free fatty acid.

### [Description of Embodiments]

The present disclosure is described hereinafter while showing the best mode thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

The definitions of the terms and/or basic technical concepts that are particularly used herein are described hereinafter when appropriate.

### (Definitions of terms)

As used herein, "lipase" refers to an enzyme, which is a type of esterase and reversibly catalyzes a reaction that hydrolyzes and decomposes neutral fat (glycerol ester) into fatty acid and glycerol. The lipase of the present disclosure is classified into triglycerol lipase, which is classified by an enzyme commission number (EC number) as EC 3.1.1.3.

Lipase activity can be determined herein by performing an enzymatic reaction using 4-nitrophenyl palmitate (4-NPP), which is an ester of palmitic acid and 4-nitrophenol, as the substrate and measuring the amount of p-nitrophenol resulting from hydrolysis of the ester at an absorbance of 410 nm. First, 4-NPP (18.9 mg) is added to 3% (v/v) Triton X-100 (12 ml) and dissolved at 70°C to prepare a substrate solution. 1 mL of the substrate solution, 0.9 mL of ion exchange water, and 1 mL of 150 mM GTA buffer (NaOH or HCl is added to 150 mM 3,3-dimethylglutaric acid, 150 mM Tris, and 150 mM 2-amino-2-methyl-1,3-propanediol and adjusted to a pH of 7.0) are placed in a cell and incubated for 5 minutes at 28°C. 0.1 mL of culture supernatant is added thereto, and the value at 410 nm is measured while agitating. Lipase activity is measured by defining the amount of enzyme producing 1 µmol of 4-nitrophenol as one unit (U), measuring the activity, and calculating units per 1 mL of culture supernatant.

The ability to decompose/consume oil and fat and fatty acid can be measured by analyzing oil and fat remaining in a medium and free fatty acid generated by hydrolysis by thin-layer chromatography. Specific quantification procedure involves firstly adding chloroform at an equal amount to the culture supernatant to extract oil and fat. 5 µl of the extract is developed on a silica gel coated plate by using a development solvent comprising chloroform, acetone, and methanol at a ratio of 96:4:1 by volume. The plate is treated with molybdic acid n-hydrate to color the oil and fat.

As used herein, "trans fatty acid-containing oil and fat" refers to oil and fat containing trans fatty acid. "Trans fatty acid" is used in the meaning that is conventionally used in the art, referring to unsaturated fatty acid with a trans double bond. Trans fatty acid is naturally present at a trace amount as conjugated linoleic acid or vaccenic acid. Trans fatty acid can be produced in a large quantity when manufacturing saturated fatty acid by hydrogenation of unsaturated fatty acid in the oil and fat industry. Food products such as margarine and shortening also contain trans fatty acid. Trans fatty acid encompasses elaidic acid, palmitelaidic acid, vaccenic acid, and the like, but the type of trans fatty acid is not particularly limited when used herein. The ratio of trans fatty acid in trans fatty acid-containing oil and fat is not particularly limited.

As used herein, "ability associated with assimilation of trans fatty acid-containing oil and fat" refers to activity resulting in assimilation of trans fatty acid-containing oil and fat by a microorganism. As used herein, "assimilate trans fatty acid-containing oil and fat" is used in the meaning that is conventionally used in the art, referring to microorganisms taking in trans fatty acid-containing oil and fat as a nutrient source such as a carbon source. "Assimilate" also includes hydrolysis into glycerol and free fatty acid as well as a change to a part of another substance.

As used herein, "ability to decompose trans fatty acid-containing oil and fat" refers to the activity to hydrolyze trans fatty acid-containing oil and fat into glycerol and free fatty acid.

As used herein, "ability to decompose oil and fat" (at each temperature) is measured as follows. Specifically, the ability is measured by purifying a lipase by the method described herein, mixing the lipase with p-nitrophenyl palmitate under a constant temperature at a temperature setting where measurements are taken, and measuring the absorbance at 410 nm. Alternatively, the ability can be measured by collecting culture supernatant of a KH-1 strain or a derivative strain thereof by the method described herein, mixing culture supernatant comprising the lipase or raw purified lipase solution with p-nitrophenyl palmitate under a constant temperature, and measuring the absorbance at 410 nm.

As used herein, "optimal temperature" for the ability to decompose oil and fat is used in the meaning that is conventionally used in the art, referring to a temperature range at which the activity to decompose oil and fat is at or above a desired constant level (refers to, for example, a temperature range at which the activity that is 80% or more of the maximum level of the enzyme is retained, but can refer to the maximum level in another embodiment). Specifically, the first lipase of the present disclosure has a peak of activity to decompose oil and fat at about 60°C, and retains 80% or more of the activity of the peak in the range of about 45 to 70°C. The second lipase of the present disclosure has a peak of activity to decompose oil at about 50°C, and retains 80% or more of the activity of the peak in the range of about 35 to 55°C. In the context of the optimal temperature of the lipase of the present disclosure, the optimal temperature of the first lipase of the present disclosure is intended to be 45 to 75°C, preferably 50 to 70°C, more preferably 55 to 65°C, still more preferably 58 to 63°C, and most preferably 60°C. The optimal temperature of the second lipase of the present disclosure is intended to be 35 to 55°C, preferably 40 to 55°C, and more preferably 45 to 50°C. In another embodiment, the lipase of the present disclosure can be altered to change the optimal temperature. The lower limit of the optimal temperature thereof can be 20°C, 25°C, 30°C, 35°C, 40°C, 45°C, 50°C, 55°C, 60°C, or the like (or a temperature therebetween that is changed in a unit of 1°C), and the upper limit can be 45°C, 50°C, 55°C, 60°C, 65°C, 70°C, 75°C, or 80°C (or a temperature therebetween that is changed in a unit of 1°C).

As used herein, "thermal stability" has the same meaning as the term "temperature stability", "heat resistance", or the like and is used in the meaning that is conventionally used in the art, referring to retention of activity by an enzyme at a high temperature. In general, an enzyme is known to be inactivated due to factors such as a change in the molecular structure at a temperature of about 50°C. In contrast, the first lipase of the present disclosure, for example, retains relative enzymatic activity, which is the enzymatic activity relative to 100% of enzymatic activity after 30 minutes of treatment at 30°C, at 100% or greater even after 30 minutes of treatment in the range of temperatures of about 30 to about 85°C, and at about 30% even after 30 minutes of treatment at 90°C, so the lipase of the present disclosure has high thermal stability. The second lipase of the present disclosure, for example, retains relative enzymatic activity, which is the enzymatic activity relative to 100% of enzymatic activity after 30 minutes of treatment at 70°C, at 90% or greater even after 30 minutes of treatment in the range of temperatures of about 15 to about 75°C, and at about 80% even at 80°C. As used herein, similar expressions such as "retains thermal stability" refer to retaining relative enzymatic activity, which is the enzymatic activity relative to 100% of enzymatic activity after 30 minutes of treatment at 30°C, at about 50% or greater.

As used herein, "optimal pH" for the ability to decompose oil and fat is used in the meaning that is conventionally used in the art, referring to a pH at which the activity to decompose oil and fat is at or above a constant level (refers to, for example, a pH range at which the activity that is 80% or more of the maximum level of the enzyme is retained, but can refer to the maximum level in another embodiment). Both the first and second lipases of the present disclosure have a peak of activity to decompose oil and fat at about pH of 9, and retain 80% or more of the peak activity in the range of about pH of 7.5 to pH of 9.5. In the context of the optimal pH of the first and second lipases, the optimal pH of the lipases (both first and second) of the present disclosure is intended to be pH of 7.5 to 9.5, preferably pH of 8.0 to 9.3, and more preferably pH of 9.

As used herein, "Burkholderia arboris" taxonomically refers to the arboris species of the genus Burkholderia. The KH-1 strain of the Burkholderia arboris species herein has a gene encoding at least two types of lipases (i.e., representative sequence of the "first lipase" and representative sequence of the "second lipase" of the present disclosure). The lipases of the present disclosure encompass, but are not limited to, those produced by a KH-1 strain of the Burkholderia arboris species or a derivative strain thereof.

As used herein, "cell-free expression system" is used in the meaning that is conventionally used in the art, referring to a system producing a recombinant protein of interest in vitro by using a transcription or translation mechanism of a biomolecule extracted from a cell. The cell-free expression system for producing the lipase of the present disclosure is not particularly limited, but a cell-free expression system derived from a prokaryote such as E. coli can be used.

As used herein, "oil treating component" refers to a component that assists in the assimilation and decomposition of oil and fat. Specific examples thereof include components that promote dispersion of oil and fat such as biosurfactants, components that decompose oil and fat into fatty acid and glycerol, components that decompose fatty acid, components that decompose glycerol, components that adsorb to and remove oil from a subject of treatment, and the like. In one aspect, an oil treating component comprises a biosurfactant produced by a KH-1 strain.

As used herein, "oil decomposing agent" refers to a formulation that is capable of decomposing oil and fat, comprising the KH-1 bacteria strain of the Burkholderia arboris species of the present disclosure, or the first or second lipase of the present disclosure produced by this bacterial strain as an active ingredient. In the present disclosure, an oil decomposing agent can be used in combination with an oil treating component. The timing of combined use of an oil decomposing agent and an oil treating component in such a case can be simultaneous use, or one can be used before the other. An oil decomposing agent can further contain a component for enhancing the activity of the bacterial strain that is used or lipase derived from the cell strain (e.g., carbon source or nitrogen source), surfactant, dry protection agent, component for maintaining bacteria for a long period of time, antiseptic, excipient, reinforcing agent, antioxidant, or the like.

The oil decomposing agent provided in the present disclosure is provided in a liquid, solid, or dried form. Examples of a liquid form include bacterial culture (can be concentrated or diluted as needed), culture supernatant, those with an enzyme component derived from culture adsorbed onto a support, those in which an enzyme is separated and purified from culture and suspended in a solvent, and the like. Examples of solid form include those suspended in a solvent comprising a protecting agent such as glycerol and frozen, those that are immobilized on a carrier (those adsorbed onto a carrier by covalent bond, electrostatic interaction, hydrophobic interaction or the like, those that are molecularly crosslinked to a carrier, and the like), those that are dehydrated by centrifugation, press compression, or the like, dried forms that have been dried, and the like. In a preferred embodiment, an oil decomposing agent is typically provided in a liquid, powder, or granule form, and can be provided with another component as a detergent or a component of a detergent.

A "derivative", "analogue", or "mutant" (of a lipase or the like) used herein preferably, although not intended to be limiting, comprises a molecule comprising a region that is substantially homologous to a target protein (e.g., lipase), and such a molecule, in various embodiments, is at least 30%, 40%, 50%, 60%, 70%, 80%, 90%, 95%, or 99% identical across the amino acid sequence of the same size or when compared to an aligned sequence that is aligned by a computer homology program known in the art, or a nucleic acid encoding such a molecule can hybridize with a sequence encoding a constituent protein under a (highly) stringent condition, moderately stringent condition, or non-stringent condition. Each of these refers to a product of altering a protein by an amino acid substitution, deletion, and addition, whose derivative is a protein that still exhibits a biological function of the original protein but not necessarily to the same degree. For example, a biological function of such a protein can be studied by a suitable and available in vitro assay that is described herein or is known in the art. As used herein, "functionally active" or "having functional activity" refers to having a structural function, regulatory function, or biochemical function of a protein such as biological activity in accordance with an embodiment associated with the polypeptide of the present disclosure, i.e., fragment or derivative.

In the present disclosure, a fragment of a lipase is a polypeptide comprising any region of a lipase, and does not necessarily have all of the biological functions of a natural lipase, as long as it functions as intended in the present disclosure (e.g., decomposition of trans fatty acid-containing oil and fat).

As used herein, "protein", "polypeptide", "oligopeptide" and "peptide" are used herein to have the same meaning and refer to a polymer of amino acids with any length. The polymer may be linear, branched, or cyclic. An amino acid may be a naturally-occurring, non-naturally-occurring, or altered amino acid. The terms may also encompass those assembled into a complex of multiple polypeptide chains. The terms also encompass naturally-occurring or artificially modified amino acid polymers. Examples of such an alteration include disulfide bond formation, glycosylation, lipidation, acetylation, phosphorylation, and any other manipulation or alteration (e.g., conjugation with a labeling component). The definition also encompasses, for example, polypeptides comprising one or more analogs of an amino acid (e.g., including non-naturally-occurring amino acids and the like), peptide-like compounds (e.g., peptoids), and other known alterations in the art. As used herein, "amino acid" is a general term for organic compounds with an amino group and a carboxyl group. When a protein or enzyme according to an embodiment of the present disclosure comprises a "specific amino acid sequence", any of the amino acids in the amino acid sequence may be chemically modified. Further, any of the amino acids in the amino acid sequence may be forming a salt or a solvate. Further, any of the amino acids in the amino acid sequence may have an L form or a D form. Even for such cases, the protein according to an embodiment of the present disclosure is considered as comprising the "specific amino acid sequence" described above. Examples of known chemical modifications applied to an amino acid comprised in a protein in vivo include modifications of the N-terminus (e.g., acetylation, myristoylation, and the like), modifications of the C-terminus (e.g., amidation, addition of glycosylphosphatidylinositol and the like), modifications of a side chain (e.g., phosphorylation, glycosylation, and the like) and the like. An amino acid may be naturally-occurring or non-naturally-occurring, as long as the objective of the present invention is met.

As used herein, "polynucleotide", "oligonucleotide", and "nucleic acid" are used in the same meaning, referring to a polymer of nucleotides of any length. The terms also encompass "oligonucleotide derivative" and "polynucleotide derivative". The "oligonucleotide derivative" and "polynucleotide derivative" are interchangeably used and refer to an oligonucleotide or polynucleotide comprising a derivative of a nucleotide or an oligonucleotide or having a bond between nucleotides that is different from ordinary bonds. Specific examples of such oligonucleotides include: 2'-O-methyl-ribonucleotide; oligonucleotide derivatives with a phosphodiester bond in an oligonucleotide converted into phosphorothioate bond; oligonucleotide derivatives with a phosphodiester bond in an oligonucleotide converted into an N3'-P5' phosphoramidate bond; oligonucleotide derivatives with a ribose and a phosphodiester bond in an oligonucleotide converted into a peptide nucleic acid bond; oligonucleotide derivatives with a uracil in an oligonucleotide substituted with a C-5 propynyl uracil; oligonucleotide derivatives with uracil in an oligonucleotide substituted with a C-5 thiazole uracil; oligonucleotide derivatives with a cytosine in an oligonucleotide substituted with a C-5 propynyl cytosine; oligonucleotide derivatives with a cytosine in an oligonucleotide substituted with a phenoxazine-modified cytosine; oligonucleotide derivatives with a ribose in DNA substituted with a 2'-O-propylribose; oligonucleotide derivatives with a ribose in an oligonucleotide substituted with a 2'-methoxyethoxy ribose; and the like. Unless noted otherwise, specific nucleic acid sequences are intended to encompass sequences that are explicitly set forth, as well as their conservatively altered variants (e.g., degenerate codon substitutes) and complementary sequences. Specifically, a degenerate codon substitute can be achieved by making a sequence in which the third position of one or more selected (or all) codons is substituted with a mixed base and/or deoxyinosine residue (Batzer et al., Nucleic Acid Res. 19: 5081 (1991); Ohtsuka et al., J. Biol. Chem. 260: 2605-2608 (1985); Rossolini et al., Mol. Cell. Probes 8: 91-98 (1994)). As used herein, "nucleic acid" is also interchangeably used with gene, cDNA, mRNA, oligonucleotide, and polynucleotide. As used herein, "nucleotide" may be naturally-occurring or non-naturally-occurring.

As used herein, "gene" refers to an agent that defines a genetic trait. A "gene" may refer to a "polynucleotide", "oligonucleotide", or "nucleic acid".

As used herein, "homology" of genes refers to the degree of identity of two or more genetic sequences with respect to one another, and having "homology" generally refers to having a high degree of identity or similarity. Therefore, the identity or similarity of sequences is higher when homology of two genes is high. Whether two types of genes have homology can be found by direct comparison of sequences or by a hybridization method under stringent conditions for nucleic acids. When two genetic sequences are directly compared, the genes are homologous typically if DNA sequences are at least 50% identical, preferably at least 70% identical, and more preferably at least 80%, 90%, 95%, 96%, 97%, 98%, or 99% identical between the genetic sequences. Thus, as used herein, "homolog" or "homologous gene product" refers to a protein in another species, preferably a microorganism, and more preferably bacteria, exerting the same biological function as a protein constituent of a complex, which will be further described herein. Such a homolog is also known as an "ortholog gene product". It is understood that such a homolog, homologous gene product, ortholog gene product, or the like can also be used, as long as they are in alignment with the objective of the present disclosure.

Amino acids may be mentioned herein by either their commonly known three letter symbols or their one character symbols recommended by the IUPAC-IUB Biochemical Nomenclature Commission. Similarly, nucleotides may be mentioned by their commonly recognized one character codes. Comparison of similarity, identity, and homology of an amino acid sequence and a base sequence is calculated herein by using a sequence analysis tool BLAST with default parameters. For example, identity can be searched using BLAST 2.7.1 (published on October 19, 2017) of the NCBI. Herein, values for identity generally refer to a value obtained when aligned under the default conditions using BLAST. However, when a higher value is obtained by changing a parameter, the highest value is considered the value of identity. When identity is evaluated in a plurality of regions, the highest value thereamong is considered the value of identity. "Similarity" is a value calculated by taking into consideration a similar amino acid in addition to identity.

In one embodiment of the present disclosure, "several" may be, for example, 15, 14, 13, 12, 11, 10, 9, 8, 7, 6, 5, 4, 3, or 2, or a value less than any one of the values. It is known that a polypeptide with one or several amino acid residue deletions, additions, insertions, or substitutions with other amino acids maintains its biological activity (Mark et al., Proc Natl Acad Sci USA. 1984 Sep; 81(18): 5662-5666., Zoller et al., Nucleic Acids Res. 1982 Oct 25; 10(20): 6487-6500., Wang et al., Science. 1984 Jun 29; 224 (4656) : 1431-1433.) A protein with a deletion or the like can be made, for example, by site-directed mutagenesis, random mutagenesis, biopanning using a protein phage library, or the like. For example, KOD-Plus-Mutagenesis Kit (TOYOBO CO., LTD.) can be used for site-directed mutagenesis. A protein with the same activity as the wildtype can be selected from mutant proteins introduced with a deletion or the like by performing various characterizations in FACS analysis, ELISA, or the like.

In one embodiment of the present disclosure, the numerical value of identity or the like, i.e., "70% or greater", can be, for example, 70% or greater, 75% or greater, 80% or greater, 85% or greater, 90% or greater, 95% or greater, 96% or greater, 97% or greater, 98% or greater, 99% or greater, or 100% or greater, or within a range between any two of numerical values of such starting points. The "identity" described above computes the ratio of the number of homologous amino acids or bases in two or more amino acid or base sequences in accordance with a known method described above. Specifically, amino acid sequences in a group of amino acid sequences to be compared are aligned before computing the ratio, and a space is introduced in a part of the amino acid sequences if needed to maximize the ratio of identical amino acids. A method for alignment, ratio computation method, comparison method, and computer program associated therewith are conventional and well known in the art (e.g., aforementioned BLAST or the like). As used herein, "identity" and "similarity" can be represented by a value measured by NCBI's BLAST, unless specifically noted otherwise. Blastp can be used with the default settings as the algorithm for comparing amino acid sequences with BLAST. Measurement results are quantified as Positives or Identities.

As used herein, "polynucleotide which hybridizes under a stringent condition" refers to commonly used, well-known conditions in the art. Such a polynucleotide can be obtained by using colony hybridization, plaque hybridization, Southern blot hybridization, or the like while using a polynucleotide selected from the polynucleotides of the present disclosure as a probe. Specifically, the polynucleotide refers to a polynucleotide that can be identified by using a filter with immobilized DNA from a colony or plaque and performing hybridization at 65°C in the presence of 0.7 to 1.0 M NaCl, and then using an SSC (saline-sodium citrate) solution with 0.1 to 2× concentration (composition of an SSC solution with 1× concentration is 150 mM sodium chloride and 15 mM sodium citrate) to wash the filter under the condition of 65°C. For "stringent condition", the following are examples of conditions that can be used. (1) low ionic strength and a high temperature are used for washing (e.g., 0.015 M sodium chloride/0.0015 M sodium citrate/0.1% sodium dodecyl sulfate at 50°C), (2) a denaturing agent such as formamide is used in hybridization (e.g., 50% (v/v) formamide, 0.1% bovine serum albumin/0.1% ficoll/0.1% polyvinyl pyrrolidone/50 mM sodium phosphate buffer with a pH of 6.5, 750 mM sodium chloride, and 75 mM sodium citrate at 42°C), or (3) a solution comprising 20% formamide, 5 × SSC, 50 mM sodium phosphate (pH 7.6), 5 × Denhardt's solution, 10% dextran sulfate, and 20 mg/ml denatured sheared salmon sperm DNA is incubated overnight at 37°C and then a filter is washed with 1 × SSC at about 37 to 50°C. The formamide concentration may be 50% or greater. Washing time can be 5, 15, 30, 60, or 120 minutes or longer. A plurality of elements such as temperature and salt concentration are conceivable as elements affecting the stringency of hybridization reactions. Ausubel et al., Current Protocols in Molecular Biology, Wiley Interscience Publishers, (1995) can be referred for details. "Highly stringent condition" is, for example, 0.0015 M sodium chloride, 0.0015 M sodium citrate, and 65-68°C or 0.015 M sodium chloride, 0.0015 M sodium citrate, 50% formamide, and 42°C. Hybridization can be performed in accordance with the method described in experimental publications such as Molecular Cloning 2nd ed., Current Protocols in Molecular Biology, Supplement 1-38, DNA Cloning 1: Core Techniques, A Practical Approach, Second Edition, Oxford University Press (1995). In this regard, a sequence comprising only an A sequence or only a T sequence is preferably excluded from a sequence that hybridizes under stringent conditions. A moderately stringent condition can be readily determined by those skilled in the art based on, for example, the length of a DNA, and is shown in Sambrook et al., Molecular Cloning: A Laboratory Manual, Third Ed., Vol. 1, 7.42-7.45 Cold Spring Harbor Laboratory Press, 2001, including, for a nitrocellulose filters, use of hybridization conditions of a pre-wash solution of 1.0 mM EDTA (pH 8.0), 0.5% SDS, and 5 × SSC, and about 50% formamide and 2 × SSC - 6 × SSC at about 40-50°C (or other similar hybridization solutions such as a Stark's solution in about 50% formamide at about 42°C) and washing conditions of 0.5 × SSC, 0.1% SDS at about 60°C. Thus, the polypeptides used in the present disclosure encompass polypeptides encoded by a nucleic acid molecule that hybridizes under highly or moderately stringent conditions to a nucleic acid molecule encoding a polypeptide described herein in particular.

The lipase of the present disclosure can be preferably "purified" or "isolated". As used herein, a "purified" substance or biological agent (e.g., nucleic acid, protein, or the like) refers to a substance or a biological agent having at least a part of an agent naturally accompanying the substance or biological agent removed. Thus, the purity of a biological agent in a purified biological agent is generally higher than the purity in the normal state of the biological agent (i.e., concentrated). The term "purified" as used herein refers to the presence of preferably at least 75% by weight, more preferably at least 85% by weight, still more preferably at least 95% by weight, and most preferably at least 98% by weight of a biological agent of the same type. The substance or biological agent used in the present disclosure is preferably a "purified" substance. An "isolated" substance or biological agent (e.g., nucleic acid, protein, or the like) as used herein refers to a substance or biological agent having an agent that naturally accompanies the substance or biological agent substantially removed. The term "isolated" as used herein varies depending on the objective. Thus, the term does not necessarily have to be represented by purity. However, when necessary, the term refers to the presence of preferably at least 75% by weight, more preferably at least 85% by weight, still more preferably at least 95% by weight, and most preferably at least 98% by weight of a biological agent of the same type. The substance used in the present disclosure is preferably an "isolated" substance or biological agent.

As used herein, "fragment" refers to a polypeptide or polynucleotide with a sequence length of 1 to n-1 with respect to the full length polypeptide or polynucleotide (with length n). The length of a fragment can be appropriately changed in accordance with the objective. Examples of the lower limit of such a length include 3, 4, 5, 6, 7, 8, 9, 10, 15, 20, 25, 30, 40, 50 and more amino acids for a polypeptide. Lengths represented by an integer that is not specifically mentioned herein (e.g., 11 and the like) can also be suitable as a lower limit. Further, examples of the length include 5, 6, 7, 8, 9, 10, 15, 20, 25, 30, 40, 50, 75, 100, and more nucleotides for a polynucleotide. Lengths represented by an integer that is not specifically mentioned herein (e.g., 11 and the like) can also be suitable as a lower limit. As used herein, such a fragment is understood to be within the scope of the present disclosure, for example when a full length version functions as an oil and fat decomposing molecule, as long as the fragment itself also functions as an oil and fat decomposing molecule.

As used herein, "biological function", in the context of a gene or a nucleic acid molecule or polypeptide related thereto, refers to a specific function that the gene, nucleic acid molecule, or polypeptide can have in vivo or ex vivo. Examples thereof include, but are not limited to, decomposition of oil and fat (e.g., decomposition of trans fatty acid-containing oil and fat), and the like. Examples thereof include, but are not limited to, functions of decomposition of trans fatty acid-containing oil and fat, decomposition of cis fatty acid-containing oil and fat, decomposition of saturated fatty acid-containing oil and fat free of a double bond, and the like in the present disclosure. As used herein, a biological function can be exerted by a corresponding "biological activity". As used herein, "biological activity" refers to activity that a certain agent (e.g., polynucleotide, protein, or the like) can have, including activity to exert a variety of functions (e.g., activity to decompose trans fatty acid-containing oil and fat). "Biological activity" can be activity exerted in vivo, or activity exerted ex vivo by secretion or the like. If an agent is for example an enzyme, the biological activity thereof encompasses the enzymatic activity thereof. Such biological activity can be measured with a technology that is well known in the art. Thus, "activity" refers to various measurable indicators that indicate or reveal the bond (either directly or indirectly) or affect a response (i.e., having a measurable effect in response to some exposure or stimulation). Examples thereof include the affinity of a compound that directly binds to the polypeptide or polynucleotide of the present disclosure, the amount of proteins upstream or downstream after some stimulation or event, and other similar scales of function.

As used herein, "expression" of a gene, a polynucleotide, a polypeptide, or the like refers to the gene or the like being subjected to a certain action in vivo to be converted into another form. Preferably, expression refers to a gene, a polynucleotide, or the like being transcribed and translated into a form of a polypeptide. However, transcription to make an mRNA is also one embodiment of expression. Thus, "expression product" as used herein encompasses such a polypeptide and protein, and mRNA. More preferably, such a polypeptide form can be a form which has undergone post-translation processing. For example, the expression level of a lipase can be determined by any method. Specifically, the expression level of a lipase can be found by evaluating the amount of mRNA of the lipase, the amount of lipase protein, and the biological activity of the lipase protein. The amount of protein or mRNA of lipase can be determined by the method described in detail in other parts of the specification or other methods known in the art.

As used herein, "functional equivalent" refers to any entity having the same function of interest but a different structure relative to the original target entity. Thus, it is understood that a functional equivalent of the "lipase" of the present disclosure encompasses mutants and variants thereof (e.g., amino acid sequence variants and the like) that are not the lipase of the present disclosure itself, which have the biological action of the lipase or can change, upon action, into a mutant or variant having the biological activity of the lipase (e.g., including nucleic acids encoding mutants and variants thereof, and vectors, cells, and the like comprising such a nucleic acid). It is understood, even without specifically mentioning, that a functional equivalent of a lipase can be used in the same manner as the lipase. The functional equivalent can be found by searching a database or the like. As used herein, "search" refers to utilizing a certain nucleic acid base sequence electronically, biologically, or by another method to find another nucleic acid base sequence having a specific function and/or property. Examples of electronic search include, but are not limited to, BLAST (Altschul et al., J. Mol. Biol. 215: 403-410 (1990)), FASTA (Pearson & Lipman, Proc. Natl. Acad. Sci., USA 85: 2444-2448 (1988)), Smith and Waterman method (Smith and Waterman, J. Mol. Biol. 147: 195-197 (1981)), Needleman and Wunsch method (Needleman and Wunsch, J. Mol. Biol. 48: 443-453 (1970)) and the like. Examples of biological search include, but are not limited to, stringent hybridization, a macroarray with a genomic DNA applied to a nylon membrane or the like or a microarray with a genomic DNA applied to a glass plate (microarray assay), PCR, in situ hybridization, and the like. Herein, a gene used in the present disclosure is intended to include corresponding genes identified by such electronic search or biological search.

As a functional equivalent of the present disclosure, it is possible to use an amino acid sequence with one or more amino acid insertions, substitutions, or deletions, or addition to one or both ends. As used herein, "one or more amino acid insertions, substitutions, or deletions, or addition to one or both ends in an amino acid sequence" refers to an alteration with a substitution of a plurality of amino acids or the like to the extent that can occur naturally by a well-known technical method such as site-directed mutagenesis or natural mutation. An altered amino acid sequence can have, for example, 1 to 30, preferably 1 to 20, more preferably 1 to 9, still more preferably 1 to 5, and especially preferably 1 to 2 amino acid insertions, substitutions, or deletions, or additions to one or both ends. Preferably, an altered amino acid sequence may be the amino acid sequence of SEQ ID NO: 4-6, 11-14, 16, or 18, having one or more (preferably 1 or several, or 1, 2, 3, 4, 5, 6, 7, 8, 9, 10, 11, 12, 13, 14, or 15) conservative substitutions. "Conservative substitution" refers herein to a substitution of one or more amino acid residues with other chemically similar amino acid residue so as not to substantially alter a function of a protein. Examples thereof include substitutions of a hydrophobic residue with another hydrophobic residue, substitutions of a polar residue with another polar residue having the same charge, and the like. Functionally similar amino acids that can be substituted in this manner are known in the art for each amino acid. Specific examples include alanine, valine, isoleucine, leucine, proline, tryptophan, phenylalanine, methionine, and the like for nonpolar (hydrophobic) amino acids, and glycine, serine, threonine, tyrosine, glutamine, asparagine, cysteine, and the like for polar (neutral) amino acids. Examples of positively charged (basic) amino acids include arginine, histidine, lysine, and the like. Further, examples of a negatively-charged (acidic) amino acid include aspartic acid, glutamic acid, and the like.

As used herein, "kit" refers to a unit providing parts to be provided (e.g., enzyme, oil and fat decomposing agent, buffer, user manual, and the like) which are generally separated into two or more segments. Such a kit form is preferred when providing a composition, which should not be provided in a mixed state for stability or the like and is preferably used by mixing immediately prior to use. Such a kit advantageously comprises an instruction or user manual describing how the provided parts (e.g., enzyme or oil and fat decomposing agent) are used or how a reagent or waste fluid after use should be processed. When a kit is used as a reagent kit herein, the kit generally comprises an instruction or the like describing the method of use of the enzyme, oil decomposing agent, or the like.

As used herein, "instruction" is a document with an explanation of the method of use of the lipase of the present disclosure for users. The instruction provides description for instructing the method of use of lipase of the present disclosure. If required, the instruction is prepared in accordance with a format specified by a regulatory authority of the country in which the present invention is practiced (e.g., Ministry of Health, Labour and Welfare, Ministry of Agriculture, Forestry and Fisheries, or the like in Japan, Food and Drug Administration (FDA) or Department of Agriculture (USDA) in the U.S., or the like), with an explicit description showing approval by the regulatory authority. An instruction can be provided in, but not limited to, paper media. An instructions can also be provided in a form such as electronic media (e.g., web sites provided on the Internet or emails).

### (Preferred embodiments>

Preferred embodiments of the present disclosure are described below. Embodiments provided below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. It is understood that the following embodiments of the present disclosure can be used alone or in combination.

### (Enzymes)

The present disclosure provides a novel polypeptide.

The present disclosure typically provides a representative sequence of a first lipase and a representative sequence of a second lipase obtained from a Burkholderia arboris species KH-1 strain or a derivative strain thereof, and derivatives thereof.

In one aspect, the polypeptide of the present disclosure can be a polypeptide, which is
(a) a polypeptide comprising the amino acid sequence set forth in SEQ ID NO: 4, 11, 16, or 18;
(b) a polypeptide having biological activity, comprising an amino acid sequence comprising one or more amino acid substitutions, additions, deletions, or a combination thereof in the amino acid sequence of (a);
(c) a polypeptide having biological activity, having at least 70% sequence identity to the amino acid sequence of (a) or (b);
(d) a polypeptide comprising an amino acid sequence encoded by the nucleic acid sequence set forth in SEQ ID NO: 1, 7, 15, or 17;
(e) a polypeptide having biological activity, encoded by a nucleic acid sequence comprising one or more nucleotide substitutions, additions, deletions, or a combination thereof in the nucleic acid sequence of (d);
(f) a polypeptide having biological activity, encoded by a nucleic acid sequence having at least 70% sequence identity to the nucleic acid sequence of (d) or (e);
(g) a polypeptide having biological activity and encoded by a nucleic acid sequence that hybridizes with a polynucleotide comprising the nucleic acid sequence of any one of (d) to (f) or a complementary sequence thereof under a stringent condition;
(h) a polypeptide having biological activity, encoded by an allelic mutant of the nucleic acid sequence of any one of (d) to (g); or
(i) a polypeptide comprising a fragment of the amino acid sequence of (a) to (h).

In another aspect, the present disclosure provides a nucleic acid sequence encoding the novel polypeptide. The polynucleotide of the present disclosure can be a polynucleotide, which is
(A) a polynucleotide comprising the nucleic acid sequence set forth in SEQ ID NO: 1, 7, 15, or 17;
(B) a polynucleotide comprising a nucleic acid sequence comprising one or more nucleotide substitutions, additions, deletions, or a combination thereof in the nucleic acid sequence of (A);
(C) a polynucleotide encoding a polypeptide having biological activity, comprising a nucleic acid sequence having at least 70% sequence identity to the nucleic acid sequence of (A) or (B);
(D) a polynucleotide encoding a polypeptide having biological activity and comprising a nucleic acid sequence that hybridizes to a polynucleotide comprising the nucleic acid of any one of (A) to (C) or a complementary sequence thereof under a stringent condition;
(E) a polynucleotide, which is an allelic mutant of the nucleic acid sequence of any one of (A) to (D), encoding a polypeptide having biological activity;
(F) a polynucleotide encoding a polypeptide comprising the amino acid sequence set forth in SEQ ID NO: 4, 11, 16, or 18;
(G) a polynucleotide encoding a polypeptide having biological activity, comprising an amino acid sequence comprising one or more amino acid substitutions, additions, deletions, or a combination thereof in the amino acid sequence of (F);
(H) a polynucleotide encoding a polypeptide having biological activity, having at least 70% sequence identity to the amino acid sequence of (F) or (G); or (I) a polynucleotide comprising a fragment of the nucleic acid sequence of (F) to (H).

The biological activity of the polypeptide of the present disclosure can be at least one of any characteristics of the first or second lipase of the present disclosure, but can comprise, for example, an ability associated with assimilation of trans fatty acid-containing oil and fat or an ability to decompose trans fatty acid-containing oil and fat, or both.

In one embodiment, the polypeptide of the present disclosure or a polypeptide encoded by a polynucleotide can thus have an ability associated with assimilation of trans fatty acid-containing oil and fat or an ability to decompose trans fatty acid-containing oil and fat, or both.

In one preferred embodiment, the polypeptide of the present disclosure or a polypeptide encoded by a polynucleotide has an ability to decompose oil and fat at 15°C or 10°C.

In another preferred embodiment, a polypeptide or polypeptide encoded by a polynucleotide, which is the first lipase of the present disclosure, has an optimal temperature for an ability to decompose oil and fat at 45 to 75°C, 50 to 70°C, preferably 55 to 65°C, 58 to 63°C, or 60°C. A polypeptide or a polypeptide encoded by a polynucleotide, which is the second lipase of the present disclosure, has an optimal temperature for an ability to decompose oil and fat at 35 to 55°C, 40 to 55°C, preferably 45 to 55°C, 45 to 50°C, or 50°C.

In one embodiment, a polypeptide or polypeptide encoded by a polynucleotide, which is the first lipase of the present disclosure, can have thermal stability at 50°C or greater, 55°C or greater, 60°C or greater, 65°C or greater, 70°C or greater, or 75°C or greater, and the thermal stability can be retained up to 50°C or less, 55°C or less, 60°C or less, 65°C or less, 70°C or less, 75°C or less, 80°C or less, 85°C or less, or 88°C or less. A polypeptide or polypeptide encoded by a polynucleotide, which is the first lipase of the present disclosure, can preferably have thermal stability at 65°C or greater, and the thermal stability can be retained up to 85°C or less. A polypeptide or polypeptide encoded by a polynucleotide, which is the second lipase of the present disclosure, can have thermal stability at 40°C or greater, 45°C or greater, 55°C or greater, 60°C or greater, 65°C or greater, 70°C or greater, or 75°C or greater, and the thermal stability can be retained up to 40°C or less, 45°C or less, 50°C or less, 55°C or less, 60°C or less, 65°C or less, 70°C or less, 75°C or less, 80°C or less, or 85°C or less. A polypeptide or polypeptide encoded by a polynucleotide, which is the second lipase of the present disclosure, can preferably have thermal stability at 50°C or greater, and the thermal stability can be retained up to 80°C or less.

In one specific embodiment, the polypeptide of the present disclosure or polypeptide encoded by a polynucleotide can be derived from, but not limited to, the Burkholderia arboris species. In a preferred embodiment, this can be an enzyme derived from, but not limited to, the Burkholderia arboris KH-1 strain or a derivative strain thereof.

In still another aspect, the present disclosure provides an oil decomposing agent comprising any of the polypeptides or cell or cell-free expression system comprising the polynucleotide.

The cell of the present disclosure comprises a polypeptide of the first or second lipase of the present disclosure, or is expressably incorporated with a polynucleotide encoding a polypeptide of the first or second lipase of the present disclosure. A cell-free expression system is provided in a manner that a polynucleotide encoding a polypeptide of the first or second lipase of the present disclosure can be expressed. A polypeptide is expressed by a suitable mechanism to exert an effect of decomposing oil.

In one embodiment, the oil decomposing agent described above comprises an additional oil treating component.

In another aspect, the present disclosure provides a kit for decomposing oil or treating oil and fat, comprising the polypeptide of the present disclosure, the cell or cell-free expression system of the present disclosure, or the oil decomposing agent, and an additional oil treating component. It is understood that an oil decomposing agent and oil treating component contained in the kit of the present disclosure of any type described elsewhere herein can be used in any combination.

In another aspect, the present disclosure provides an oil decomposing and removing method comprising causing the polypeptide of the present disclosure, the cell or cell-free expression system of the present disclosure, or the oil decomposing agent of the present disclosure to act on a subject of treatment. In the oil decomposing and removing method of the present disclosure, the subject of treatment preferably comprises trans fatty acid-containing oil and fat, but the subject is not limited thereto. The present specification shows that oil can be very efficiently decomposed by using the lipase of the present disclosure, even for a subject free of trans fatty acid-containing oil and fat.

In another aspect, the present disclosure provides a detergent comprising the polypeptide of the present disclosure, the cell or cell-free expression system of the present disclosure, or the oil decomposing agent of the present disclosure.

In still another aspect, application using the polypeptide of the present disclosure, the cell or cell-free expression system of the present disclosure, or the oil decomposing agent of the present disclosure to technologies for fat modification or oil and fat production (transesterification or the like) is provided. Examples of technologies for fat modification or oil and fat production (transesterification or the like) include a reaction generating an ester form such as ethyl ester or methyl ester, substitution reaction in oil and fat-containing fatty acid, production of diacylglycerol or monoacyl glycerol, and the like.

The polypeptide of the present disclosure is intended to encompass not only polypeptides having the amino acid sequence of SEQ ID NO: 4-6, 11-14, 16, or 18, but also variants thereof. Examples of such a polypeptide include polypeptide having biological activity, comprising an amino acid sequence comprising one or more amino acid substitutions, additions, deletions, or a combination thereof in the amino acid sequence of SEQ ID NO: 4-6, 11-14, 16, or 18. In a specific embodiment, amino acids corresponding to positions 1, 3, 6, 137, 220, 227, 243, 276, and 316 in the amino acid sequence set forth in SEQ ID NO: 4 are substituted in a representative sequence of the first lipase of the invention. In another embodiment, amino acids corresponding to positions 13, 26, 45, 75, 100, 138, 168, 171, 214, 230, 234, 248, 250, 331, and 360 in the amino acid sequence set forth in SEQ ID NO: 11 are substituted in a representative sequence of the second lipase of the invention.

The present disclosure further provides a nucleic acid sequence encoding the novel polypeptide. The nucleic acid sequence is intended to encompass not only nucleic acid sequences set forth in SEQ ID NO: 1-3, 7-10, 15, or 17, but also variants thereof. Examples of such a nucleic acid sequence include nucleic acid sequences encoding a polypeptide having biological activity, comprising one or more nucleotide substitutions, additions, deletions, or a combination thereof in the nucleic acid sequence of SEQ ID NO: 1-3, 7-10, 15, or 17.

The position where a mutation can be introduced in an amino acid sequence or nucleic acid sequence described above can be readily determined by those skilled in the art by methodologies such as homology search, motif search, domain analysis, alignment, or secondary structure prediction. The position where a mutation can be introduced in an amino acid sequence or nucleic acid sequence can be any position, or mutations can be introduced at a plurality of positions, as long as a variant having a mutation at such a position retains the activity of the lipase of the present disclosure. Specifically, a mutation can be at any position of SEQ ID NO: 6 (full length amino acid sequence) for the first lipase of the present disclosure. As one example, a mutation at a residue other than the residues corresponding to S at position 131, D at position 308, and H at position 330 of SEQ ID NO: 6 is desirable, but a mutation is not limited thereto.

### (Production of enzyme)

The first or second lipase of the present disclosure can be purified from, for example, a Burkholderia arboris species KH-1 strain (accession number NITE BP-02731) or a derivative strain thereof. Specifically, the first lipase of the present disclosure can be purified by a method comprising:
(a) culturing a KH-1 strain or a derivative strain thereof for 24 hours or longer at 28°C in a medium comprising 1% canola oil;
(b) sterilizing the culture supernatant of (a) using a filter with 0.45 µm pores and applying the culture supernatant to a Butyl-S Sepharose 6 Fast Flow (GE Healthcare) that has been equilibriated in advance with about 10 column volumes of 20 mM Tris-HCl (pH 7.0) buffer (hereinafter, referred to as Tris buffer) comprising 0.5 M NaCl and 2 mM CaCl₂, and leaving the culture supernatant standing for about 1 hour to allow a hydrophobic protein to adsorb to a column;
(c) washing the column of (b) with about 10 column volumes of Tris buffer, and then washing the column 5 times with about 1 column volume of NaCl-free Tris buffer, and allowing a protein that is weakly bound to a column carrier to elute; and
(d) washing the column of (c) 4 times with about 1 column volume of Tris buffer comprising 0.5% Triton X-100 and allowing a protein that is strongly bound to the column to elute.

The second lipase of the present disclosure can be purified by a method comprising:
(a) culturing a KH-1 strain or a derivative strain thereof for 48 hours or longer at 15°C in a medium comprising 1% canola oil;
(b) sterilizing the culture supernatant of (a) using a filter with 0.45 µm pores and applying the culture supernatant to a Butyl-S Sepharose 6 Fast Flow (GE Healthcare) that has been equilibriated in advance with about 10 column volumes of 20 mM Tris-HCl (pH 7.0) buffer (hereinafter, referred to as Tris buffer) comprising 0.5 M NaCl and 2 mM CaCl₂, and leaving the culture supernatant standing for about 1 hour to allow a hydrophobic protein to adsorb to a column;
(c) washing the column of (b) with about 10 column volumes of Tris buffer, and then washing the column 5 times with about 1 column volume of NaCl-free Tris buffer, and allowing a protein that is weakly bound to a column carrier to elute; and
(d) washing the column of (c) 4 times with about 1 column volume of Tris buffer comprising 0.5% Triton X-100 and allowing a protein that is strongly bound to the column to elute.

The detailed conditions such as the conditions for culturing a KH-1 strain or a derivative strain thereof and column chromatography conditions in an enzyme purification method are appropriately adjusted by those skilled in the art.

The first and second lipases of the present disclosure can also be produced by a secretory production system using microorganisms. Specifically, the first or second lipase of the present disclosure can be produced by a method comprising:
(a)introducing a nucleic acid molecule (e.g., SEQ ID NO: 1, 7, 15, or 17 or a variant sequence thereof) encoding a mature form of the first or second lipase of the present disclosure into a pBIC1 plasmid (Takara Bio) and allowing the expression thereof within Brevibacillus bacteria; and (b) purifying a protein secreted from the Brevibacillus bacteria with Ni-Sepharose.

The lipase recombinant proteins of the present disclosure can also be produced with an expression system such as a CORYNEX® system (Ajinomoto) using Corynebacterium glutamicum, Pichia pastoris expression system (Thermo Fisher), Baculovirus expression system (Thermo Fisher) using insect cells, protein secretory expression system (Ozeki) using koji bacteria, or a pET system based E. coli periplasm secretory production system (Merck). Detailed conditions such as a method of expressing a lipase, a host for expressing a lipase, and a purification method of lipase secreted from a host in such a lipase production method using microorganisms are appropriately adjusted by those skilled in the art. Furthermore, the lipase of the present disclosure can also be expressed using various expression systems such as intrabacterial expression systems and cell-free expression systems in addition to microorganism secretory production systems.

### (Use of enzymes)

The lipase of the present disclosure is useful in the treatment of oil and fat and can be used in the treatment of wastewater and liquid waste comprising oil and fat or the like. In this embodiment, the lipase of the present disclosure is used in wastewater treatment. The lipase of the present disclosure used in wastewater treatment can be used in applications such as factory wastewater, kitchen wastewater, and household wastewater.

In another example, the lipase of the present disclosure is used as a detergent. The lipase of the present disclosure used as a detergent can be used in applications such as laundry detergents, kitchen detergents, cleaning detergents, and industrial detergents. A detergent comprising the lipase of the present disclosure is particularly useful as a drainage system detergent such as a pipe cleaner.

In another embodiment, the lipase of the present disclosure is used in technologies for fat modification or oil and fat production. The lipase of the present disclosure used in technologies for fat modification or oil and fat production can be used in food, industrial, fuel, and other applications.

In another embodiment, the lipase of the present disclosure can be used as measures for environmental contamination. The lipase of the present disclosure used as measures for environmental contamination can be used for removing contaminants in soil contamination, ground water contamination, ocean contamination or the like due to oil.

In another embodiment, the lipase of the present disclosure is used in waste treatment and composting. The lipase of the present disclosure used in waste treatment and composting can be used in applications such as food waste treatment including biodecomposition, composting, feed preparation from agricultural products/food waste, and reduction of volume of oily sludge produced by a dissolved air flotation apparatus or a grease trap.

In another embodiment, the lipase of the present disclosure is used as a pharmaceutical product. The lipase of the present disclosure used as a pharmaceutical product can be used in applications such as digestion agents and fat decomposition promoting agents.

In another embodiment, the lipase of the present disclosure is used as a cosmetic product. The lipase of the present disclosure used as a cosmetic product can be used in applications such as cosmetic products for improving, preventing, or treating oily skin.

The lipase of the present disclosure can be used as a component comprising an isolated enzyme and as a component comprising the bacteria themselves.

### (General technology)

The molecular biological methodology, biochemical methodology, and microbiological methodology used herein are well known and conventionally used in the art, which are described, for example, in Savli, H., Karadenizli, A., Kolayli, F., Gundes, S., Ozbek, U., Vahaboglu, H. 2003. Expression stability of six housekeeping genes: A proposal for resistance gene quantification studies of Pseudomonas aeruginosa by real-time quantitative RT-PCR. J. Med. Microbiol. 52: 403-408., Marie-Ange Teste, Manon Duquenne, Jean M Francois and Jean-Luc Parrou 2009. Validation of reference genes for quantitative expression analysis by real-time RT-PCR in Saccharomyces cerevisiae. BMC Molecular Biology 10: 99, Seiji Ishii, Hiroshi Okumura, Chiyo Matsubara, Fumi Ninomiya, Hiroshi Yoshioka, 2004, "Netsukannousei Polima wo Mochiita Suichuyubun no Kani Sokutei Hoho [Simple method of measuring oil-in-water content using heat sensitive polymer]", Vol 46, No.12, "Journal of water and waste", or the like. Relevant portions thereof (which may be the entire document) are incorporated herein by reference.

### (Note)

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments and Examples specifically described herein and is limited only by the scope of claims.

### [Examples]

The Examples are described hereinafter. When required, organisms used in the following Examples were handled in compliance with the guidelines stipulated by the Nagoya University, regulatory agency, or the Cartagena Protocol. For reagents, the specific products described in the Examples were used. However, the reagents can be substituted with an equivalent product from another manufacturer (Sigma-Aldrich, Fuji Film, Wako Pure Chemical, Nacalai Tesque, R & D Systems, USCN Life Science INC, Thermo Fisher Scientific, Kanto Chemical, Funakoshi, Tokyo Chemical Industry, Merck, or the like).

### (Example 1: Analysis on expression of estimated lipase)

This Example shows analysis on expression of an estimated lipase gene found in a KH-1 strain at each temperature.

### (Experimental methodology)

A KH-1 strain was inoculated into 3L of an inorganic salt medium (Na₂HPO₄ at 3.5 g/L, KH₂PO₄ at 2.0 g/L, (NH₄)₂SO₄ at 4.0 g/L, MgCl₂·6H_{2O} at 0.34 g/L, FeSO₄·7H₂O at 2.8 mg/L, MnSO₄·O5H₂O at 2.4 mg/L, CoCl₂·6H₂O at 2.4 mg/L, CaCl₂·2H₂O at 1.7 mg/L, CuCl₂·2H₂O at 0.2 mg/L, ZnSO₄·7H₂O at 0.3 mg/L, and NaMoO₄ at 0.25 mg/L) comprising 1% canola oil so that the final concentration according to bacterial optical density would be OD₆₆₀ = 0.03 by using a HITACHI U-2810 spectrophotometer (Hitachi, Tokyo, Japan), and cultured in a 5L volume fermenter (250 rpm, under 200 ml/min air circulation) at 28°C or 15°C. Total RNA was extracted using a High Pure RNA Isolation Kit (Roche) from each culture sampled over time. cDNA was synthesized by removing genomic DNA using Prime Script™ RT reagent Kit with gDNA Eraser Perfect Real Time (Takara Bio), with 2 µg of the total RNA as a template. The undiluted cDNA solution was then diluted 3-fold using a dilution solution that was part of a kit. Real-time quantitative RT-PCR was performed with Applied Biosystems StepOnePlus™ (Applied Biosystems) using a synthetic primer specific to a gene encoding the first lipase and the second lipase of the present disclosure. A PCR reaction was performed in a 20 µl solution comprising PowerUp™ SYBR® Green Master Mix (Thermo Fisher Scientific) (10 µl), each primer (final concentration of 0.5 µM), and cDNA (1 µl). The PCR reaction was performed with a program that performs 1 cycle of denaturation for 2 minutes at 95°C and then repeats a cycle of 3 seconds at 95°C and 30 seconds at 60°C 40 times, by using a fast cycling mode. The expression levels were normalized with the expression level of an RNA polymerase sigma factor (rpoD). After confirming that the melting curve has a single peak, data was analyzed by comparative Ct method (ΔΔCt method). The relative expression levels after setting each lipase gene expressed when cultured in an LB medium to 1 as a control are shown.

### (Results)

Culture of a KH-1 strain in the presence of oil and fat was found to induce expression of a gene encoding two types of lipases (first lipase with a representative sequence and second lipase with a representative sequence). As shown in the following Table 1, a gene encoding the first lipase with a representative sequence exhibited a peak expression level at about 24 hours when cultured at 28°C, and exhibited a peak expression level at about 60 hours when cultured at 15°C. A gene encoding the second lipase with a representative sequence exhibited a peak expression level at about 24 hours when cultured at 28°C, and exhibited a peak expression level at about 96 hours when cultured at 15°C.

### [Table 1]

**Table 1. The amount of expression of gene encoding the lipase of the present invention in a canola oil-containing inorganic salt medium at each culture temperature (relative value after setting the amount of expression in an LB medium to 1)**

| | First lipase | Second lipase |
|---|---|---|
| 28°C | 136 ± 39 (24 hours) | 198 ± 26 (24 hours) |
| 15°C | 57 ± 12 (60 hours) | 134 ± 28 (96 hours) |

The time within the parenthesis is the culture time at which the expression level exhibited a peak.

### (Example 2: Experiment on mutants)

This Example shows an experiment using a mutant of the lipase of the present disclosure.

### (Experimental methodology)

Polypeptides introduced with a mutation to an amino acid residue other than those at sites known as an active site in a lipase so that sequence identity would be 70 to 99% in each of the amino acid sequence (SEQ ID NO: 4) of the first lipase of the present disclosure with a representative sequence and the amino acid sequence (SEQ ID NO: 11) of the second lipase of the present disclosure with a representative sequence are prepared. The activity to decompose trans fatty acid-containing oil and fat is measured using the mutant polypeptides.

### (Example 3: Induction of expression of the first and second lipases of the present disclosure with trans fatty acid and trans fatty acid-containing oil and fat)

This Example compared the expression levels of genes encoding the first and second lipases of the present disclosure with a representative sequence upon addition of trans fatty acid (elaidic acid) and trans fatty acid-containing oil and fat (trielaidin) with those for cis fatty acid (oleic acid) and cis fatty acid-containing fatty acid (triolein).

### (Experimental methodology)

A KH-1 strain was cultured for 2 days in a triolein plate. The KH-1 strain was then scraped off with a cotton swab, suspended in PBS, and then washed twice with PBS. A Triton X-100 solution with a final concentration of 5% comprising oleic acid, triolein, elaidic acid, or trielaidin with a final concentration of 2% was prepared as a stock solution of oil and fat and dissolved at 70°C. To 20 ml of an inorganic salt medium, a stock solution of each oil and fat was added so that the ratio of the amount would be 1/10 by volume, and the washed KH-1 strain was inoculated thereto (final concentration: 0.2% for oil and fat, 0.5% for Triton X-100; bacterial strain: OD₆₆₀ = 0.1). After culturing for 6 hours at 28°C, the KH-1 strain was harvested, and total RNA was extracted from the harvested KH-1 strain. After synthesizing cDNA in accordance with the above description by using 2 µg of the total RNA as a template, quantitative RT-PCR was performed using cDNA diluted 3-fold as a template. The expression levels were compared as relative values obtained from normalizing the levels with the amount of expression of an rpoD gene, and setting the expression level in culture with oleic acid or triolein to 1.

### (Results and discussion)

The expression level of a gene encoding the first lipase with a representative sequence increased about 100-fold after trielaidin treatment (Figures 1A and 1B on the top row). This result suggests that trans fatty acid or trans fatty acid-containing oil and fat can be an agent inducing the first lipase. The expression of a gene coding the second lipase with a representative sequence also increased about 10- to 20-fold after trielaidin treatment (Figures **1A** and **1B** on the bottom row). This result suggests that trans fatty acid or trans fatty acid-containing oil and fat can also be an agent inducing the second lipase.

### (Example 4: Purification of the first lipase from supernatant of KH-1 strain cultured at 28°C)

This Example shows purification of the first lipase with a representative sequence from KH-1 strain culture supernatant at 28°C.

### (Experimental methodology)

4 ml of Butyl-S Sepharose 6 Fast Flow (GE Healthcare) was equilibriated with 40 ml of 20 mM Tris-HCl (pH 7.0) buffer comprising 0.5 M NaCl and 2 mM CaCl₂. The culture supernatant after culturing a KH-1 strain at 28°C was sterilized with a 0.45 µm filter, and applied to a column. After leaving the supernatant standing for 1 hour and allowing a hydrophobic protein to adsorb, the column was washed with 40 ml of the aforementioned buffer, and subsequently washed 5 times with 4 ml of the aforementioned buffer that is free of NaCl to allow a protein that was weakly bound to a column carrier to elute out. The column was further washed 4 times with 4 ml of the aforementioned buffer comprising 0.5% Triton X-100 to allow a protein strongly bound to a column carrier by a hydrophobic bond to elute out. Each eluted sample was filtered with a 0.45 µm filter. To 20 µl thereof, 4 µl of 5× SDS-PAGE sample buffer was added. After heat treatment for 5 minutes at 100°C, the sample was centrifuged, and 20 µl of supernatant was applied to a polyacrylamide gel (5 to 12% gradient gel) to perform electrophoresis for 90 minutes at 20 mA. The proteins contained in the samples were then separated and analyzed by applying CBB staining.

The N-terminal amino acid sequence of the eluted lipase was analyzed. After concentrating the eluted fraction in a spin column with a 30 kDa cut-off, the sample was applied to a surfactant removing column DetergentOut™ (TaKaRa), which is a spin format column, for replacement with 20 mM Tris-HCl (pH 7.0) buffer comprising 2 mM CaCl₂ in accordance with the manufacturer's protocol. Triton X-100 used upon elution was removed. The resulting lipase sample without surfactants was separated by SDS-PAGE. After blotting the protein of interest on a PVDF membrane, the N-terminal amino acid sequence was determined by Edman degradation.

### (Results)

Proteins purified from the culture supernatant at 28°C exhibited a molecular weight of about 30 kDa (Figure 2). The lipase purified by hydrophobic column chromatography from the culture supernatant of KH-1 at 28°C was shown to be nearly a single band, consisting of nearly a single protein.

The N-terminal sequence of the proteins purified from the culture supernatant at 28°C was decoded as Ala-Asp-Asn-Tyr-Ala and confirmed to be a product of a gene encoding the first lipase.

### (Discussion)

The amino acid sequence of a mature protein of the first lipase with a representative sequence matches the sequence from position 45 of a product estimated from the genetic sequence obtained from the genetic information in RAST, suggesting that the amino acid residues at positions 1 to 44 are cleaved after secretion from a cell as pre-sequence.

### (Example 5: Purification of the second lipase from supernatant of KH-1 strain cultured at 15°C)

This Examples shows purification of the second lipase with a representative sequence from KH-1 strain culture supernatant at 15°C.

### (Experimental methodology)

A lipase was purified from culture supernatant after culturing a KH-1 strain at 15°C by the same methodology as Example 4. After separating purified protein fractions by SDS-PAGE, a band of interest was cut out from gel, bleached, and washed. Tris buffer (pH 8.0) comprising trypsin was added, and the solution was subjected to enzymatic digestion for 20 hours at 35°C. The recovered sample solution was desalinated/concentrated and then analyzed by LC-MS/MS (Thermo Fisher Scientific Inc., USA).

### (Results)

Proteins purified from the culture supernatant at 15°C exhibited a molecular weight of about 40 kDa (Figure 3). The lipase purified by hydrophobic column chromatography from the culture supernatant of KH-1 at 15°C was shown to be nearly a single band, consisting of nearly a single protein.

A fragment of Asn-Val-Thr-Tyr-His was detected, while a sequence closer to the N-terminus side was not detected from the result of mass spectrometry on the proteins purified from the culture supernatant at 15°C. It was inferred in view of the specificity of trypsin digestion that this is a product of a gene encoding the second lipase whose N-terminus sequence is this sequence, or Thr-Arg-Asn-Val-Thr-Tyr-His, which is this sequence extended further by two residues to the N-terminus side.

### (Discussion)

The amino acid sequence of a mature protein of the second lipase with a representative sequence matches the sequence from position 49 or 51 of a product estimated from the genetic sequence obtained from the genetic information in RAST, suggesting that the amino acid residues at positions 1 to 48 or 50 are cleaved after secretion from a cell as pre-sequence.

### (Example 6: Thermal stability, optimal temperature, optimal pH, and pH stability of the lipase of the present disclosure)

This Example shows the thermal stability, optimal temperature, optimal pH, and pH stability of the first and second lipases of the present disclosure.

### (Experimental methodology)

The first and second lipases of the present disclosure with a representative sequence were purified by hydrophobic column chromatography from a KH-1 strain cultured at 28°C and 15°C, respectively, and adjusted to 5 U/ml. Each lipase activity was quantified by measuring hydrolysis of p-nitrophenyl palmitate under the following conditions (A) to (D). (A) Buffer adjusted to a temperature between 10 to 80°C and the first or second lipase of the present disclosure with a representative sequence were mixed, and lipase activity was measured immediately thereafter at each temperature. (B) The first or second lipase of the present disclosure was incubated for 30 minutes at a temperature between 30 to 95°C, and then each lipase activity was measured at 37°C. (C) Acetic acid buffer (pH 3.0 to 5.0), sodium phosphate buffer (pH 5.0 to 7.0), Tris-HCl buffer (pH 7.0 to 9.0), or CAPS buffer (pH 9.0 to 11.0) was mixed with the first or second lipase of the present disclosure with a representative sequence, and then lipase activity was measured. The lipase activity is shown as a relative strength after setting the lipase activity at pH of 9.0 to 100%. (D) Each buffer used in (C) and a solution of the first or second lipase of the present disclosure with a representative sequence were mixed at a ratio of 1:1 by volume, left standing for 24 hours at 28°C, and then lipase activity was measured at pH of 7.0.

### (Results)

The first lipase of the present disclosure with a representative sequence had an optimal temperature at 60°C and exhibited thermal stability in a broad temperature range between 30°C to 85°C (Figure **4**). The second lipase of the present disclosure with a representative sequence had an optimal temperature at 50°C and exhibited thermal stability in a broad temperature range between 15°C to 80°C (Figure **5**). The optimal pH of the first lipase of the present disclosure with a representative sequence was pH of 9.0, and stable pH thereof was pH of 9.0 to 9.5. The optimal pH of the second lipase of the present disclosure with a representative sequence was pH of 9.0, and stable pH thereof was pH of 7.5 to 10.5.

### (Discussion)

The results of optimal temperature and thermal stability of the first and second lipases of the present disclosure demonstrated that the lipases of the present disclosure are lipases retaining high activity even at a high temperature.

### (Example 7: Comparison of ventilator stain decomposition activities between KH-1 stain culture supernatant and detergents)

This Example compared ventilator stain decomposition activities of KH-1 stain culture supernatant with those of a detergent for oil and multipurpose detergent.

### (Experimental methodology)

As the KH-1 strain culture supernatant, supernatant of culture from culturing a KH-1 strain for 24 hours at 28°C in a medium comprising 1% canola oil was used. As the detergent for oil and multipurpose detergent, natural enzyme detergent Nicoeco for kitchen (Nicoeco, Nagano, Japan, diluted with water 143-fold in accordance with the instruction manual) and Family® (Kao, Tokyo, Japan, diluted 666-fold in accordance with the instruction manual) were used, respectively. A ventilator filter with an oil stain deposit was cut into 2 cm squares and placed in a plate. 5 ml of KH-1 strain culture supernatant (KH-1), detergent for oil, or multipurpose detergent was added to each plate, and the filter was soaked for 30 minutes to 4 hours.

### (Results)

Figure **6** shows the results of Example 7. Oil stains could not be completely removed even after 4 hours of soak washing with the multipurpose detergent or detergent for oil, but the filter was cleaned to the same degree as a new product by soak washing for 1 hour with the KH-1 strain culture supernatant.

### (Discussion)

Since it is understood that the primary lipase in the culture supernatant obtained under such culture conditions is the enzyme of the present disclosure, it is understood that the oil stain decomposition activity is attributed to the enzyme of the present disclosure.

### (Example 8: Comparison of ventilator stain decomposition activities of the enzymes of the present disclosure derived from KH-1 strain and N-51032 enzyme)

This Example compared the ventilator stain decomposition activities of the first and second lipases of the present disclosure with a representative sequence derived from a KH-1 strain with those of Novozym 51032 lipase (Novozymes).

### (Experimental methodology)

The first and second lipases of the present disclosure with a representative sequence derived from a KH-1 strain were each obtained by purifying culture supernatant of the KH-1 strain by hydrophobic column chromatography in accordance with the above descriptions. Novozym 51032 lipase (N-51032) was purchased from Novozymes. Each lipase was dissolved in 20 mM Tris-HCl buffer (pH of 7.4) comprising 2 mM CaCl₂ and 0.25% Triton X-100 so that the concentration would be 15 U/ml. A ventilator filter with an oil stain deposit was cut into 2 cm squares and placed in a plate. 5 ml of each lipase solution was added to each plate, and the filter was soaked for 30 minutes.

### (Results)

Figure **7** shows a ventilator filter after being soaked in a lipase solution and a lipase solution after soaking. Oil stains on each of the ventilator filters soaked in the first and second lipases of the present disclosure with a representative sequence were decomposed significantly more than those on the ventilator filter soaked in Novozym 51032 lipase.

### (Example 9: Decomposition of lard and shortening by the first lipase of the present disclosure)

This Example shows lard and shortening decomposition activities due to the first lipase of the present disclosure with a representative sequence.

### (Experimental methodology)

The first lipase of the present disclosure with a representative sequence was purified from culture supernatant of a KH-1 strain by hydrophobic column chromatography using 20 mM Tris-HCl (pH of 7.0) elution buffer comprising 2 mM CaCl₂ and 0.5% Triton X-100. 2 ml of a solution of the lipase of the present disclosure (50 U/ml) was placed on a plate, and 0.7 g of lard (A) or 0.5 g of shortening (B) was added thereto, which was left standing for 24 hours at 28°C while agitating as appropriate to allow them to blend with the solution. Lard and shortening treated only with the aforementioned elution buffer was used as a control.

(C) A stock solution was prepared by dissolving lard or shortening in 2.5% Triton X-100 so that the final concentration would be 2%. 0.2 ml of the stock solution in which the lard and shortening were melted at 65°C was added to 1.8 ml of the lipase containing buffer described above. The final concentrations of Triton X-100 and each oil and fat were 0.25% and 0.2%, respectively. The mixture was incubated for 24 hours at 28°C. Oil and fat was extracted with an equal amount of chloroform, and 5 µl of the extract was subjected to thin-layer chromatography.

### (Results)

Figure **8** shows the results of Example 9. In view of the experimental results in this Example, the lard and shortening treated with the buffer alone were still in a solid state after the treatment, whereas oil and fat treated with the first lipase with a representative sequence was dissolved (Figures **8(A)** and **(B)**). The results of thin-layer chromatography demonstrated that triglyceride in the lard and shortening was decomposed into free fatty acid (Figure **8(C)**). The first lipase of the present disclosure was demonstrated to hydrolyze lard and shortening and have solubilizing activities.

### (Example 10: Decomposition of lard and shortening by the second lipase of the present disclosure)

This Example shows lard and shortening decomposition activities due to the second lipase of the present disclosure.

### (Experimental methodology)

The second lipase of the present disclosure with a representative sequence was purified from culture supernatant of a KH-1 strain cultured for 96 hours at 15°C by hydrophobic column chromatography using 20 mM Tris-HCl (pH of 7.0) elution buffer comprising 2 mM CaCl₂ and 0.5% Triton X-100. 2 ml of a solution of the lipase of the present disclosure (50 U/ml) was placed on a plate, and 0.7 g of lard (A) or 0.5 g of shortening (B) was added thereto, which was left standing for 24 hours at 28°C while agitating as appropriate to allow them to blend with the solution. Lard and shortening treated only with the aforementioned elution buffer was used as a control.

(C) A stock solution was prepared by dissolving lard or shortening in 2.5% Triton X-100 so that the final concentration would be 2%. 0.2 ml of the stock solution in which the lard and shortening were melted at 65°C was added to 1.8 ml of the lipase containing buffer described above. The final concentrations of Triton X-100 and each oil and fat were 0.25% and 0.2%, respectively. The mixture was incubated for 24 hours at 28°C. Oil and fat was extracted with an equal amount of chloroform, and 5 µl of the extract was subjected to thin-layer chromatography.

### (Results)

Figure **9** shows the results of Example 10. In view of the experimental results in this Example, the lard and shortening treated with the buffer alone were still in a solid state after the treatment, whereas oil and fat treated with the second lipase with a representative sequence was dissolved (Figures **9(A)** and **(B)**). The results of thin-layer chromatography demonstrated that triglyceride in the lard and shortening was decomposed into free fatty acid (Figure **9(C)**). The second lipase of the present disclosure with a representative sequence was demonstrated to hydrolyze lard and shortening and have solubilizing activities.

### (Example 11: Test for treating trans fatty acid-containing wastewater with KH-1 strain)

This Example shows a test for treating trans fatty acid-containing wastewater with a lipase of a KH-1 strain and BioRemove 3200 (BR3200, Novozymes).

### (Experimental methodology)

(A) N (nitrogen) and P (phosphorous) corresponding to an inorganic salt medium were added to a wastewater sample. The KH-1 strain was cultured in an LB medium, then washed, inoculated so as to be OD = 0.1 and subjected to conditions for secreting a lipase. BR3200 (BioRemove 3200, Novozymes) was added at an amount that is 10-fold of the concentration that is generally used. The wastewater sample was cultured for 24 or 48 hours at 28°C. Subsequently, the sample was developed on a silica gel coated plate using a development solvent comprising chloroform, acetone, and methanol at a ratio of 96:4:1 by volume. Oil and fat decomposition was detected by coloring with molybdic acid n-hydrate by using thin-layer chromatography.
(B) The oil content concentration in terms of a normal hexane value in the wastewater samples after 24 or 48 hour treatment was measured with a measuring reagent kit.

### (Results)

It was found that a lipase secreted by a KH-1 strain can decompose oil content in a wastewater sample by 24 hour treatment to about 50% of the level for BR3200 (Figure **10****).** Furthermore, a lipase secreted by a KH-1 strain was able to decompose oil content in a wastewater sample by 48 hour treatment to about 1/7 of the level for BR3200.

### (Example 12: Decomposition of each oil and fat by the first lipase of the present disclosure)

This Example shows decomposition of triolein and trielaidin by the first lipase of the present disclosure with a representative sequence.

### (Experimental methodology)

The first lipase of the present disclosure with a representative sequence was purified by hydrophobic column chromatography from culture supernatant after culturing a KH-1 strain at 28°C in an inorganic salt medium comprising 1% canola oil and used. Triolein and trielaidin were dissolved into distilled water comprising 5% Triton X-100 so that the final concentration would be 2% as the stock solution (10× stock solution). 100 µl of each of a solution of the first lipase of the present disclosure with a representative sequence (in 20 mM Tris buffer (comprising 2 mM CaCl₂ and 0.5% Triton X-100; pH of 7.0) with a final concentration of 30 U/ml)) and the same buffer free of lipase was dispensed into a tube, and the oil and fat 10× stock solution described above was added to a ratio of 1/10 by volume. The final concentration of each oil and fat was 0.2%, and the final concentration of Triton X-100 was 0.5%. These solutions were incubated for 24 hours at 37°C. Subsequently, the solutions were developed on a silica gel coated plate using a development solvent comprising chloroform, acetone, and methanol at a ratio of 96:4:1 by volume. Oil and fat decomposition was detected by coloring with molybdic acid n-hydrate by using thin-layer chromatography.

### (Results)

The first lipase of the present disclosure with a representative sequence had the activity to hydrolize both triolein and trielaidin (Figure **11**)**.**

### (Discussion)

The first lipase of the present disclosure with a representative sequence was found to have activity to hydrolyze triolein, which is a cis triglyceride, and trielaidin, which is a trans triglyceride.

### (Example 13: Decomposition of each oil and fat by the second lipase of the present disclosure)

This Example shows decomposition of triolein and trielaidin by the second lipase of the present disclosure with a representative sequence.

### (Experimental methodology)

The second lipase of the present disclosure with a representative sequence was purified by hydrophobic column chromatography from culture supernatant after culturing a KH-1 strain at 15°C in an inorganic salt medium comprising 1% canola oil and used. 100 µl of each of a solution of the second lipase of the present disclosure (in 20 mM Tris buffer (comprising 2 mM CaCl₂ and 0.5% Triton X-100; pH of 7.0) with a final concentration of 0.3 U/ml)) and the same buffer free of lipase was dispensed into a tube, and the oil and fat 10× stock solution described above was added to a ratio of 1/10 by volume. The final concentration of each oil and fat was 0.2%, and the final concentration of Triton X-100 was 0.5%. These solutions were incubated for 22 hours at 37°C. Subsequently, the solutions were developed on a silica gel coated plate using a development solvent comprising chloroform, acetone, and methanol at a ratio of 96:4:1 by volume. Oil and fat decomposition was detected by coloring with molybdic acid n-hydrate by using thin-layer chromatography.

### (Results)

The second lipase of the present disclosure with a representative sequence had the activity to hydrolize both triolein, which is a cis triglyceride, and trielaidin, which is a trans triglyceride (Figure **12**).

### (Example 14: Decomposition of each oil and fat by the lipases of the present disclosure at a low temperature)

This Example shows decomposition of trioliein and trielaidin by the first and second lipases of the present disclosure with a representative sequence under a 15°C condition.

### (Experimental methodology)

The first lipase with a representative sequence was purified by hydrophobic column chromatography from culture supernatant after culturing a KH-1 strain at 28°C in an inorganic salt medium comprising 1% canola oil and used. The second lipase with a representative sequence was purified by hydrophobic column chromatography from culture supernatant after culturing a KH-1 strain at 15°C in the same manner and used. Triolein or trielaidin was mixed with the first or second lipase solution by adding the 10× stock solution described above at 1/20 so that the final concentration would be 0.1%. The first lipase with a representative sequence was adjusted to be a final concentration of 6 U/ml against 4-NPP as the substrate, and the second lipase with a representative sequence was adjusted to be a final concentration of 6 U/ml against 4-NPP as the substrate. These solutions were incubated for 72 hours at 15°C. Subsequently, the solutions were developed on a silica gel coated plate using a development solvent comprising chloroform, acetone, and methanol at a ratio of 96:4:1 by volume. Oil and fat decomposition was detected by coloring with molybdic acid n-hydrate by using thin-layer chromatography.

### (Results and discussion)

Triolein, which is cis triglyceride, was decomposed into oleic acid by treatment with either the first or second lipase with a representative sequence. Trielaidin, which is trans triglyceride, was decomposed into elaidic acid by treatment with either the first or second lipase (Figure **13**)**.** These results demonstrate that the first and second lipases of the present disclosure with a representative sequence have activity to decompose both cis fatty acid-containing oil and fat and trans fatty acid-containing oil and fat under a low temperature (15°C) condition.

### (Example 15: Oil and fat technologies for fat modification and fatty acid methyl ester production using triolein)

This Example shows fat modification of cis fatty acid by the first and second lipases of the invention with a representative sequence.

### (Experimental method)

A KH-1 strain was cultured in a fermenter for 48 hours in a BS culture medium comprising 1% canola oil. From the supernatant thereof, the first and second lipases with a representative sequence were purified using a Butyl Sepharose column. The solutions of the first and second lipases were adjusted to 5 U/ml. Triolein was dissolved in methanol to be 0.5% as a stock solution. A mixture of 80 µl of the stock solution and 20 µl of each enzyme solution was dispensed in a tube with a screw cap and left standing for 96 hours in an incubator at 37°C. The solution mixed with lipase-free buffer was used as a control. Subsequently, 100 µl of water and 50 µl of chloroform were added to each solution and thoroughly agitated with a vortex mixer, and then centrifuged for 5 minutes at 12000 g. 10 µl of the bottom layer of the chloroform layer was subjected to TLC analysis using a development solvent comprising hexane, diethyl ether, and acetic acid at a ratio of 80:20:1 by volume.

### (Result)

Figure **14** shows the results of Example 15. Both the first and second lipases of the invention catalyzed a transesterification reaction of triolein, which is a cis triglyceride, and generated methyl oleate ester.

### (Example 16: Oil and fat technology for fat modification and fatty acid methyl ester production using trans fatty acid)

This Example shows fat modification of trans fatty acid by the first and second lipases of the invention with a representative sequence.

### (Experimental method)

A KH-1 strain was cultured in a fermenter for 48 hours in a BS culture medium comprising 1% canola oil. From the supernatant thereof, the first and second lipases with a representative sequence were purified using a Butyl Sepharose column. The solutions of the first and second lipases were adjusted to 5 U/ml. Each of the trans fatty acid monomers, (A) palmitelaidic acid and (B) vaccenic acid, was dissolved in methanol such that each fatty acid monomer would be 0.5% as a stock solution. A mixture of 80 µl of each trans fatty acid stock solution and 20 µl of each enzyme solution was dispensed in a tube with a screw cap and left standing for 72 hours in an incubator at 37°C. The solution mixed with lipase-free buffer was used as a control. Subsequently, 100 µl of water and 50 µl of chloroform were added to each solution and thoroughly agitated with a vortex mixer, and then centrifuged for 5 minutes at 12000 g. 5 µl of the bottom layer of the chloroform layer was subjected to TLC analysis using a development solvent comprising hexane, diethyl ether, and acetic acid at a ratio of 80:20:1 by volume.

### (Result)

Figure **15** shows the results of Example 16. Both the first and second lipases of the invention catalyzed a transesterification reaction of palmitelaidic acid and vaccenic acid, which are trans fatty acid, and generated methyl palmitelaidate ester and methyl vaccenate ester, respectively. This revealed that these enzymes are also useful in a reaction of a trans fatty acid that is not elaidic acid.

### (Example 17: Oil and fat decomposition activity of a variant of the first lipase of the invention)

This Example analyzed the oil and fat decomposition activity of a variant of the first lipase of the invention.

### (Experimental method)

An expression construct of a variant (SEQ ID NO: 16) prepared from substituting amino acids corresponding to positions 1, 3, 6, 137, 220, 227, 243, 276, and 316 with serine, aspartic acid, threonine, serine, valine, threonine, leucine, glutamine, and lysine, respectively, in the amino acid sequence of the first lipase of the invention with a representative sequence (SEQ ID NO: 4) was constructed in accordance with an expression system (BIC system: TaKaRa) using a Brevibacillus expression system. Bacterial cells were cultured at 30°C for 48 hours in a medium prepared by adding neomycin to a 2SY medium so that the final concentration would be 50 µg/ml. 50 ml of the supernatant of the cultured bacteria was applied to a Butyl Sepharose column and eluted to 20 mM Tris-HCl comprising 0.25% Triton X-100 and 2 mM CaCl₂. A recombinant lipase has AD+6xHis+DDDK (Enterokinase recognition sequence) added to the N-terminus of the mature sequence. Triolein (TOle) and trielaidin (TED) were used as oil and fat. Decomposition activity of each oil and fat was analyzed by TLC in accordance with the following experimental conditions:
*Type of oil and fat: triolein, trielaidin
*Reaction solution: 20 mM Tris-HCl (pH 7.0), 2 mM CaCl₂, 0.5% Triton X100
*Final concentration of oil and fat: 0.2%
*Treatment method: 100 µl of enzyme solution was placed in an Eppendorf tube, and 10× each oil and fat stock was added at 1/5 of the amount.
*Treatment temperature: 37°C
*Treatment time: 48 hours
*Oil content extraction: oil content was extracted with half the amount of chloroform and 10 µl was applied to perform TLC.
*Development plate: silica gel coated plate
*Development solvent: chloroform:acetone:methanol = 96:4:2
*Detection: coloring by molybdic acid n-hydrate (2.4 g/60 ml EtOH)

### (Results)

Figure **16** shows the results of Example 17. A variant with mutations at the 9 positions described above also had high oil and fat decomposition activity.

### (Example 18: Oil and fat decomposition activity of a variant of the second lipase of the invention)

This Example analyzed the oil and fat decomposition activity of a variant of the second lipase of the invention.

### (Experimental method)

An expression construct of a variant (SEQ ID NO: 18) prepared from substituting amino acids at positions 13, 26, 45, 75, 100, 138, 168, 171, 214, 230, 234, 248, 250, 331, and 360 with leucine, leucine, arginine, isoleucine, valine, serine, glutamic acid, arginine, glycine, asparagine, serine, arginine, glycine, asparagine, and alanine, respectively, in the amino acid sequence of the second lipase of the invention with a representative sequence (SEQ ID NO: 11) was constructed in accordance with an expression system (BIC system: TaKaRa) using a Brevibacillus expression system. Bacterial cells were cultured at 30°C for 72 hours in a medium prepared by adding MgCl₂ and neomycin to a TM medium so that the final concentrations would be 20 mM and 50 µg/ml, respectively. 50 ml of the supernatant of the cultured bacteria was applied to a Butyl Sepharose column and eluted to 20 mM Tris-HCl comprising 0.25% Triton X-100 and 2 mM CaCl₂. A recombinant lipase has AD+6xHis+DDDK (Enterokinase recognition sequence) added to the N-terminus of the sequence where the estimated signal sequence was cleaved. Triolein (TOle) and trielaidin (TED) were used as oil and fat. Decomposition activity of each oil and fat was analyzed by TLC in accordance with the following experimental conditions:
*Type of oil and fat: triolein, trielaidin
*Reaction solution: 20 mM Tris-HCl (pH 7.0), 2 mM CaCl₂, 0.5% Triton X100
*Final concentration of oil and fat: 0.2%
*Treatment method: 100 µl of enzyme solution was placed in an Eppendorf tube, and 10× each oil and fat stock was added at 1/5 of the amount.
*Treatment temperature: 37°C
*Treatment time: 48 hours
*Oil content extraction: oil content was extracted with an equal amount of chloroform and 10 µl was applied to perform TLC.
*Development plate: silica gel coated plate
*Development solvent: chloroform:acetone:methanol = 96:4:2
*Detection: coloring by molybdic acid n-hydrate (2.4 g/60 ml EtOH)

### (Results)

Figure **17** shows the results of Example 18. A variant with mutations at 15 positions described above also had high oil and fat decomposition activity.

### (Note)

As described above, the present disclosure is exemplified by the use of its preferred embodiments. However, it is understood that the scope of the present disclosure should be interpreted solely based on the Claims. It is also understood that any patent, any patent application, and any references cited herein should be incorporated herein by reference in the same manner as the contents are specifically described herein. The present application claims priority to Japanese Patent Application No. 2018-129504 filed on July 6, 2018 with the Japan Patent Office. It is understood that the entire content thereof is incorporated herein by reference.

### [Industrial Applicability]

The present disclosure is useful in the treatment of trans fatty acid-containing wastewater which is an issue at food factories and the like by decomposing trans fatty acid-containing wastewater.

### [Reference to Deposited Biological Material]

NITE BP-02731

### [Sequence Listing Free Text]

SEQ ID NO: 1 mature sequence of a representative nucleic acid sequence of the first lipase of the present disclosure SEQ ID NO: 2 nucleic acid sequence comprising a nucleotide encoding a pre-sequence in a representative nucleic acid sequence of the first lipase of the present disclosure
SEQ ID NO: 3 full length sequence of a representative nucleic acid sequence of the first lipase of the present disclosure
SEQ ID NO: 4 mature sequence of a representative amino sequence of the first lipase of the present disclosure SEQ ID NO: 5 amino acid sequence comprising a pre-sequence in a representative amino acid sequence of the first lipase of the present disclosure
SEQ ID NO: 6 full length sequence of a representative amino acid sequence of the first lipase of the present disclosure
SEQ ID NO: 7 mature sequence of a representative nucleic acid sequence of the second lipase of the present disclosure
SEQ ID NO: 8 nucleic acid sequence comprising a nucleotide encoding a short chain pre-sequence in a representative nucleic acid sequence of the second lipase of the present disclosure
SEQ ID NO: 9 nucleic acid sequence comprising a nucleotide encoding a long chain pre-sequence in a representative nucleic acid sequence of the second lipase of the present disclosure
SEQ ID NO: 10 full length sequence of a representative nucleic acid sequence of the second lipase of the present disclosure
SEQ ID NO: 11 mature sequence of a representative amino sequence of the second lipase of the present disclosure SEQ ID NO: 12 amino acid sequence comprising a short chain pre-sequence in a representative amino acid sequence of the second lipase of the present disclosure SEQ ID NO: 13 amino acid sequence comprising a long chain pre-sequence in a representative amino acid sequence of the second lipase of the present disclosure
SEQ ID NO: 14 full length sequence of a representative amino acid sequence of the second lipase of the present disclosure
SEQ ID NO: 15 variant sequence of a mature sequence of a representative nucleic acid sequence of the first lipase of the present disclosure
SEQ ID NO: 16 variant sequence of a mature sequence of a representative amino acid sequence of the first lipase of the present disclosure
SEQ ID NO: 17 variant sequence of a mature sequence of a representative nucleic acid sequence of the second lipase of the present disclosure
SEQ ID NO: 18 variant sequence of a mature sequence of a representative amino acid sequence of the second lipase of the present disclosure

## Claims

1. A polypeptide, which is
(a) a polypeptide comprising the amino acid sequence set forth in SEQ ID NO: 4, 11, 16, or 18;
(b) a polypeptide having biological activity, comprising an amino acid sequence comprising one or more amino acid substitutions, additions, deletions, or a combination thereof in the amino acid sequence of (a);
(c) a polypeptide having biological activity, having at least 70% sequence identity to the amino acid sequence of (a) or (b);
(d) a polypeptide comprising an amino acid sequence encoded by the nucleic acid sequence set forth in SEQ ID NO: 1, 7, 15, or 17;
(e) a polypeptide having biological activity, encoded by a nucleic acid sequence comprising one or more nucleotide substitutions, additions, deletions, or a combination thereof in the nucleic acid sequence of (d);
(f) a polypeptide having biological activity, encoded by a nucleic acid sequence having at least 70% sequence identity to the nucleic acid sequence of (d) or (e);
(g) a polypeptide having biological activity and encoded by a nucleic acid sequence that hybridizes with a polynucleotide comprising the nucleic acid sequence of any one of (d) to (f) or a complementary sequence thereof under a stringent condition;
(h) a polypeptide having biological activity, encoded by an allelic mutant of the nucleic acid sequence of any one of (d) to (g); or
(i) a polypeptide comprising a fragment of the amino acid sequence of (a) to (h).

2. The polypeptide of claim 1, wherein the biological activity is an ability associated with assimilation of trans fatty acid-containing oil and fat or an ability to decompose trans fatty acid-containing oil and fat.

3. The polypeptide of claim 1 or 2, wherein 45 to 70°C is an optimal temperature for an ability to decompose oil and fat.

4. The polypeptide of claim 1 or 2, wherein 35 to 55°C is an optimal temperature for an ability to decompose oil and fat.

5. The polypeptide of any one of claims 1 to 3, having thermal stability at 65°C or greater.

6. The polypeptide of any one of claims 1, 2, and 4, having thermal stability at 75°C or greater.

7. The polypeptide of any one of claims 1 to 6, having an ability to decompose oil and fat at 15°C.

8. The polypeptide of any one of claims 1 to 7, which is a polypeptide derived from Burkholderia arboris.

9. A polynucleotide, which is
(A) a polynucleotide comprising the nucleic acid sequence set forth in SEQ ID NO: 1, 7, 15, or 17;
(B) a polynucleotide comprising a nucleic acid sequence comprising one or more nucleotide substitutions, additions, deletions, or a combination thereof in the nucleic acid sequence of (A);
(C) a polynucleotide encoding a polypeptide having biological activity, comprising a nucleic acid sequence having at least 70% sequence identity to the nucleic acid sequence of (A) or (B);
(D) a polynucleotide encoding a polypeptide having biological activity and comprising a nucleic acid sequence that hybridizes with a polynucleotide comprising the nucleic acid sequence of any one of (A) to (C) or a complementary sequence thereof under a stringent condition;
(E) a polynucleotide, which is an allelic mutant of the nucleic acid sequence of any one of (A) to (D), encoding a polypeptide having biological activity;
(F) a polynucleotide encoding a polypeptide comprising the amino acid sequence set forth in SEQ ID NO: 4, 11, 16, or 18;
(G) a polynucleotide encoding a polypeptide having biological activity, comprising an amino acid sequence comprising one or more amino acid substitutions, additions, deletions, or a combination thereof in the amino acid sequence of (F);
(H) a polynucleotide encoding a polypeptide having biological activity, having at least 70% sequence identity to the amino acid sequence of (F) or (G); or
(I) a polynucleotide comprising a fragment of the nucleic acid sequence of (F) to (H).

10. The polynucleotide of claim 9, wherein the biological activity is an ability associated with assimilation of trans fatty acid-containing oil and fat or an ability to decompose trans fatty acid-containing oil and fat.

11. The polynucleotide of claim 9 or 10, encoding a polypeptide for which 45 to 70°C is an optimal temperature for an ability to decompose oil and fat.

12. The polynucleotide of claim 9 or 10, encoding a polypeptide for which 35 to 55°C is an optimal temperature for an ability to decompose oil and fat.

13. The polynucleotide of any one of claims 9 to 11, encoding a polypeptide having thermal stability at 65°C or greater.

14. The polynucleotide of any one of claims 9, 10, or 12, encoding a polypeptide having thermal stability at 75°C or greater.

15. The polynucleotide of any one of claims 9 to 14, wherein the polynucleotide encodes a polypeptide having an ability to decompose oil and fat at 15°C.

16. The polynucleotide of any one of claims 9 to 15, which is a polynucleotide derived from Burkholderia arboris.

17. A cell or a cell-free expression system comprising the polynucleotide of any one of claims 9 to 16.

18. An oil decomposing agent comprising the polypeptide of any one of claims 1 to 8, a cell or a cell-free expression system comprising the polynucleotide of any one of claims 9 to 16, or the cell or cell-free expression system of claim 17.

19. The oil decomposing agent of claim 18, comprising an additional oil treating component.

20. A kit for decomposing oil, comprising the polypeptide of any one of claims 1 to 8, a cell or a cell-free expression system comprising the polynucleotide of any one of claims 9 to 16, the cell or cell-free expression system of claim 17, or the oil decomposing agent of claim 18, and an additional oil treating component.

21. An oil decomposing and removing method comprising causing the polypeptide of any one of claims 1 to 8, a cell or a cell-free expression system comprising the polynucleotide of any one of claims 9 to 16, the cell or cell-free expression system of claim 17, or the oil decomposing agent of claim 18 or 19 to act on a subject of treatment.

22. The method of claim 21, wherein the subject of treatment comprises trans fatty acid or trans fatty acid-containing oil and fat.

23. A detergent comprising the polypeptide of any one of claims 1 to 8, a cell or a cell-free expression system comprising the polynucleotide of any one of claims 9 to 16, or the cell or cell-free expression system of claim 17.

24. Use of the polypeptide of any one of claims 1 to 8, a cell or a cell-free expression system comprising the polynucleotide of any one of claims 9 to 16, the cell or cell-free expression system of claim 17, or the oil decomposing agent of claim 18 or 19 in a technology for fat modification or oil and fat production.
